# EUROPEAN PATENT APPLICATION

(11) **EP 4 471 426 A2**
(43) Date of publication of application: **04.12.2024**
(21) Application number: 24178923.9
(22) Date of filing: 29.05.2024
(51) Int. Cl.: G01N 33/564, G01N 33/68

(54) **METHOD AND REAGENTS FOR THE DETECTION OF A MAMMALIAN ANTIBODY**

(30) Priority: 01.06.2023 EP 23176874
(71) Applicant: EUROIMMUN Medizinische Labordiagnostika AG, 23560 Lübeck (DE); Klinisch-immunologisches Labor Prof. h.c. (RCH) Dr. med. Winfried Stöcker, 23627 Groß Grönau (DE)
(72) Inventor: SCHARF, Madeleine, 23560 Lübeck (DE); MISKE, Ramona, 23560 Lübeck (DE); RADZIMSKI, Christiane, 23560 Lübeck (DE); SCHÖBEL, Stefan, 23627 Gross Grönau (DE); STÖCKER, Winfried, 23627 Gross Grönau (DE); BOROWSKI, Kathrin, 23627 Gross Grönau (DE); CORTY, Merle, 23627 Gross Grönau (DE)

(57) **Abstract**

The present invention relates to a method comprising the step detecting in a sample a mammalian antibody binding specifically to ZSCAN5, a mammalian antibody binding specifically to ZSCAN5, a carrier with a solid phase with at least one immobilized polypeptide comprising at least one epitope of ZSCAN5 or a variant thereof and a use of the mammalian antibody for diagnosing a neurological autoimmune disease or a cancer.

## Description

The present invention relates to a method comprising the step detecting in a sample a mammalian antibody binding specifically to ZSCAN5, a mammalian antibody binding specifically to ZSCAN5, a carrier with a solid phase with at least one polypeptide comprising at least one epitope of ZSCAN5 or a variant thereof and a use of the mammalian antibody for diagnosing a neurological autoimmune disease or a cancer.

Neurological autoimmune diseases are rare but potentially treatable. Their hallmark is the occurrence of neurological symptoms and an autoimmune response, usually associated with the emergence of an antibody binding specifically to a structure in the brain such as a polypeptide having important functions for neurotransmission. They may affect any area of the nervous system, including the central, peripheral and autonomic nervous system. Although the system involvement is often multifocal, like encephalomyelitis, it may involve a single system, e.g. cerebellar degeneration.

The neurological autoimmune disease may be associated with cancer. If that is the case, it may be referred to as PNS (paraneoplastic syndrome). The neurological symptoms precede or follow the cancer diagnosis, although, in some cases, the primary cancer is not found even at autopsy. Most of the PNS reflect a nervous system-specific autoimmune attack initiated by onconeural antigens released to the peripheral lymphoid tissue from an unsuspected primary or recurrent neoplasm. Frequently, a cerebrospinal fluid (CSF) study in these patients reveals lymphocytic pleocytosis, elevated protein, increased IgG synthesis and oligoclonal bands, supporting the immunological pathology. Antibodies binding specifically to an accessible membrane target are directly responsible for the disease, as in the case of acetyl choline receptor antibodies in myasthenia gravis and P/Q type voltage-gated calcium channels in Lambert Eaton Myasthenic syndrome. Often, the cancer is asymptomatic at the time of presentation with neurological syndrome. Some of the paraneoplastic antibodies are specifically associated with cancer and some are not.

The notion that neuropsychiatric symptoms including recurrent seizures and impairment of cognition and behavior are linked to distinct autoantibodies has fundamentally improved the diagnostic and therapeutic approaches to several severe neurological disorders, including the disease spectrum of encephalitis, including limbic encephalitis (LE) and autoimmune encephalitis. Autoantibodies binding specifically to neuronal surface proteins such as NMDAR, AMPAR, mGluR5, GABABR, DPPX or LGI1 and onconeural targets including amphiphysin, BMPER and PNMA2 and GAD65 targeting intracellular protein structures have been found to be associated with encephalitis.

Early diagnosis and treatment of neurological autoimmune diseases is important because any delay can result in rapid progression and irreversible neurological damage. By contrast, early and appropriate treatment, usually by some form of immunosuppressive therapy, may reverse some of the damages, and, in some cases, even a complete recovery is possible. For example, 80% of patients suffering from NMDA receptor (NMDAR) encephalitis have a good outcome following immunosuppressive treatment, although long-term mental problems or a recurrence are possible.

Diagnosing neurological autoimmune diseases is often difficult, though. One of the reasons is the absence of a particular clinical pattern of symptoms and the absence of specific imaging and laboratory abnormalities. For example, despite the progress in recognizing autoantibody mediated immune mechanisms in a substantial proportion (> 50%) of patients with encephalitis, in particular suspected LE, specific 'neurological' antibodies are not detectable. About 70% of encephalitis cases with unclear etiology remain without definite diagnosis even after extensive evaluation for infectious etiologies. A better understanding of immunological mechanisms in seronegative encephalitis patients may open new therapy options for affected individuals.

Treatment of underlying cancer, if present, is important in the treatment of the neurological condition and often paramount for the patient's survival rate. However, 30-40% of suspected or putative PNS patients will not present with any antibody detectable by state-of-the-art test because many diagnostically relevant autoantibodies are still unknown. A high risk remains that these patients remain undiagnosed or even misdiagnosed.

The present invention is based on the inventors' surprising finding that mammalian antibodies to ZSCAN5 exist and can be detected in samples from patients suffering from a neurological autoimmune disease and/or cancer, but not in samples from healthy subjects.

Therefore, the problem underlying the present invention is to provide reagents and methods for the diagnosis and treatment of an autoimmune disease, such as a neurological autoimmune disease, and/or cancer, preferably associated with the presence of a mammalian antibody binding specifically to ZSCAN5 and for the manufacture of diagnostic and therapeutic products.

Another problem underlying the present invention is to distinguish an autoimmune disease caused by a mammalian antibody binding specifically to ZSCAN5 and a disease caused by other factors, in particular an infection. Further factors causing a disease, which can be distinguished based on the present invention from an autoimmune disease, include alcohol or drug abuse and psychiatric conditions not caused by the presence of the inventive antibodies.

Another problem underlying the present invention is to provide an assay with an increased diagnostic reliability, in particular with regard to specificity and/or sensitivity, for the diagnosis for an autoimmune disease, such as a neurological autoimmune disease, or cancer, preferably PNS and/or LE, optionally in combination with state-of-the-art assays.

The problem underlying the present invention is solved by the subject-matter of the attached independent and dependent claims.

In a first aspect, the problem underlying the present invention is solved by a method comprising the step detecting in a sample a mammalian antibody binding specifically to ZSCAN5.

In a second aspect, the problem underlying the present invention is solved by a method for isolating a mammalian antibody binding specifically to ZSCAN5, comprising the steps
a) Immobilizing on a carrier at least one polypeptide comprising at least one epitope of ZSCAN5 or a variant thereof,
b) contacting a sample comprising antibodies with the at least one polypeptide under conditions compatible with formation of a complex, wherein said antibody binds to said polypeptide,
c) separating the complex formed in step a) from the sample,
d) optionally separating the antibody from the polypeptide, and
e) optionally detecting the antibody or complex.

In a third aspect, the problem underlying the present invention is solved by a method comprising the steps
contacting a polypeptide comprising at least one epitope of ZSCAN5 or a variant thereof with a liquid comprising a mammalian antibody binding specifically to ZSCAN5, wherein a candidate drug is present and/or the liquid does not comprise a sample from a subject to be diagnosed, and
detecting binding of the antibody, preferably in quantitative manner.

In a preferred embodiment, the method comprises the step immobilizing the polypeptide comprising at least one epitope of ZSCAN5 or a variant thereof on a carrier, preferably prior to contacting the polypeptide with the liquid. In another preferred embodiment, the candidate drug is present in the liquid and/or is contacted with the antibody prior to contacting the polypeptide with the liquid.

In a fourth aspect, the problem is solved by a mammalian antibody binding specifically to ZSCAN5, preferably in a liquid comprising one or more, more preferably all from the group comprising an artificial buffer, a preservative and an artificial anticoagulant and/or from the group comprising blood such as whole blood, plasma, serum and CSF, optionally wherein the liquid is diluted.

In a fifth aspect, the problem is solved by providing a carrier, preferably a diagnostically useful carrier, comprising a solid phase comprising at least one polypeptide, preferably immobilized, comprising at least one epitope of ZSCAN5 or a variant thereof and a) a negative and/or positive control and/or b) at least one additional antigen, preferably diagnostically useful antigen, preferably wherein the at least one polypeptide and the negative and/or positive control or additional antigen are spatially separate on the carrier, or a first carrier, preferably a first diagnostically useful carrier, with a solid phase comprising at least one polypeptide, preferably immobilized, comprising at least one epitope of ZSCAN5 or a variant thereof, and a second carrier, preferably a second diagnostically useful carrier, comprising a solid phase comprising an immobilized a) negative and/or positive control and/or b) at least one additional polypeptide comprising an immobilized antigen or a variant thereof.

In a preferred embodiment, at least one additional antigen is at least one antigen, preferably all antigens from the group consisting of NMDAR, Lgl1, AMPA1, AMPA2, CASPR2, GABA B, GABA A, DPPX, IGLON5, Hu, Yo, CV2/CRMP5, Ri, Ma2, Amphiphysin, Recoverin, RGS8, DAGLA, STX1B, AK5, AP3B2, Flotillin1+2, GRM1, GRM2, GRM5, GLURD2, ITPR1, KCNA2, NCDN, Septin 3+5+6+7+11 and Sez6L2, or a variant thereof.

In a sixth aspect, the problem is solved by a kit comprising at least one polypeptide comprising at least one epitope of ZSCAN5 or a variant thereof and a carrier, preferably a diagnostically useful carrier, more preferably the carrier according to the invention, wherein
a) the at least one polypeptide is immobilized on a carrier, preferably a diagnostically useful carrier, more preferably the carrier according to the present invention, and the kit further comprises a means for detecting a mammalian antibody, preferably binding specifically to ZSCAN5,
b) the at least one polypeptide and the carrier are configured for immobilizing the at least one polypeptide on the surface of the carrier, preferably via an affinity tag and a ligand binding to the affinity tag, and the kit further comprises a means for detecting a mammalian antibody, preferably binding specifically to ZSCAN5,
c) the carrier is immobilized with a means for capturing an antibody and the kit further comprises a means for detecting a mammalian antibody, preferably the at least one polypeptide comprising a detectable label, or
d) the carrier and a means for capturing an antibody are configured for immobilizing the means for capturing an antibody on the surface of the carrier, preferably via an affinity tag and a ligand binding to the affinity tag, and the kit further comprises a means for detecting a mammalian antibody, preferably the at least one polypeptide comprising a detectable label,
and preferably one or more, more preferably all from the group consisting of a recombinant antibody binding to ZSCAN5, a mammalian antibody binding to ZSCAN5, a chemical solution reactive with a detectable label, a positive control, a negative control, a water-tight vessel for incubating a sample with a carrier or reagent, a sample dilution buffer, a wash buffer, and a calibrator, preferably a set of calibrators.

In a 7^{th} aspect, the problem is solved by a use of a) a mammalian antibody binding specifically to ZSCAN5 or a variant thereof, or b) the combination of at least one polypeptide comprising at least one epitope of ZSCAN5 or a variant thereof and a means for detecting a mammalian antibody, or c) the carrier of the invention, or d) the kit of the invention for identifying a patient having an increased risk of suffering from or developing a disease associated with the presence of a mammalian antibody binding specifically to ZSCAN5, and/or for diagnosing a neurological autoimmune disease or a cancer.

In an 8^{th} aspect, the problem is solved by a use of at least one polypeptide comprising at least one epitope of ZSCAN5 or a variant thereof, or a mammalian antibody binding specifically to ZSCAN5 or a variant thereof, or a recombinant antibody binding specifically to ZSCAN5 or a variant thereof for the manufacture of a kit, preferably analytical kit, or medical device, preferably diagnostic device, optionally for the diagnosis of a disease or for aiding in the diagnosis of a disease.

In a 9^{th} aspect, the problem is solved by a use of a mammalian antibody binding specifically to ZSCAN5 or a variant thereof, or a recombinant antibody binding specifically to ZSCAN5 or a variant thereof, wherein the antibody is preferably recognized by secondary antibodies to human IgG class immunoglobulins, as a positive control for the detection of an antibody binding specifically to ZSCAN5 or a variant thereof in a sample.

In a 10^{th} aspect, the problem is solved by an ex *vivo* method for removing an antibody binding specifically to ZSCAN5 or a variant thereof from blood, preferably blood of a mammalian patient, or preferably serum of a mammalian patient.

In an 11^{th} aspect, the problem is solved by a method for diagnosing a disease or for aiding in the diagnosis of a disease, such as an autoimmune disease, preferably a neurological autoimmune disease, associated with a mammalian antibody binding specifically to ZSCAN5 or a variant thereof. In one embodiment, the method for diagnosing a disease or for aiding in the diagnosis of a disease is an *in vivo* method. In one embodiment, the method for diagnosing a disease or for aiding in the diagnosis of a disease is an ex *vivo* method. In one embodiment, the method for diagnosing a disease or for aiding in the diagnosis of a disease comprises using at least one polypeptide comprising at least one epitope of ZSCAN5 or a variant thereof. In one embodiment, the disease is an autoimmune disease, preferably a neurological autoimmune disease, such as those described herein, and/or a cancer, such as those described herein. In one embodiment, the method for diagnosing a disease or for aiding in the diagnosis of a disease comprises the step detecting a mammalian antibody binding specifically to ZSCAN5 or a variant thereof from blood, preferably blood of a mammalian patient, or preferably serum of a patient.

In one embodiment, ZSCAN5 comprises at least one Zinc finger domain and at least one SCAN oligomerization domain. In a preferred embodiment, ZSCAN5 comprises one or more, preferably all amino acid sequences selected from the group comprising SEQ ID NO:1, SEQ ID NO:2, SEQ ID NO:3 and SEQ ID NO:4 or a variant thereof. In one embodiment, ZSCAN5 comprises 1, 2, 3 or 4 amino acid sequences selected from the group comprising SEQ ID NO:1, SEQ ID NO:2, SEQ ID NO:3 and SEQ ID NO:4 or a variant thereof. Preferably, ZSCAN5 is represented by ZSCAN5A, ZSCANB and/or ZSCAN5C. In one embodiment, ZSCAN5 is represented by ZSCAN5A, wherein the protein optionally comprises an amino acid sequence as set forth in SEQ ID NO:5 or a variant thereof. In one embodiment, ZSCAN5 is ZSCAN5B, wherein the protein optionally comprises an amino acid sequence as set forth in SEQ ID NO:6 or a variant thereof. In one embodiment, ZSCAN5 is ZSCAN5C, wherein the protein optionally comprises an amino acid sequence as set forth in SEQ ID NO:7 or a variant thereof. Preferably, ZSCAN5 comprises the amino acid sequence as set forth in SEQ ID NO:8 or a variant thereof. In one embodiment, ZSCAN5 comprises one or more amino acid sequences selected from the group comprising SEQ ID NO:9, SEQ ID NO:10, SEQ ID NO:11, SEQ ID NO:12, SEQ ID NO: 13, SEQ ID NO:14, SEQ ID NO:15, SEQ ID NO:16 and SEQ ID NO:17 or a variant thereof. In one embodiment, ZSCAN5 comprises one or more amino acid sequences selected from the group comprising SEQ ID NO:18, SEQ ID NO:19 and SEQ ID NO:20 or a variant thereof. In one embodiment, ZSCAN5 comprises one or more amino acid sequences selected from the group comprising SEQ ID NO:21, SEQ ID NO:22, SEQ ID NO:23 and SEQ ID NO:24 or a variant thereof.

In one embodiment, a mammalian antibody binding to ZSCAN5 binds to ZSCAN5A or a variant thereof. In one embodiment, a mammalian antibody binding to ZSCAN5 binds to ZSCAN5B or a variant thereof. In one embodiment, a mammalian antibody binding to ZSCAN5 binds to ZSCAN5C or a variant thereof. In one embodiment, a mammalian antibody binding to ZSCAN5 binds to ZSCAN5A or a variant thereof and/or ZSCAN5B or a variant thereof and/or ZSCAN5C or a variant thereof. In a preferred embodiment, an antibody binding to ZSCAN5 or a variant thereof binds to ZSCAN5A or a variant thereof but not to ZSCAN5B or ZSCAN5C. In another preferred embodiment, an antibody binding to ZSCAN5 or a variant thereof binds to ZSCAN5B or a variant thereof but not to ZSCAN5A or ZSCAN5C. In another preferred embodiment, an antibody binding to ZSCAN5 or a variant thereof binds to ZSCAN5C or a variant thereof but not to ZSCAN5A or ZSCAN5B. In another preferred embodiment, an antibody binding to ZSCAN5 or a variant thereof binds to all three forms of ZSCAN5 or variants thereof.

In a preferred embodiment, the polypeptide comprising at least one epitope of ZSCAN5 comprises the amino acid sequence as set forth in SEQ ID NO:8 or a variant thereof.

In a preferred embodiment, the autoimmune disease is a neurological autoimmune disease, preferably associated with the presence of a mammalian antibody specifically binding to ZSCAN5 or a variant thereof. In a further preferred embodiment, the autoimmune disease, such as the neurological autoimmune disease, is selected from the group consisting of paraneoplastic neurological syndrome (PNS), dementia, cognitive decline, psychotic symptoms, acoustic hallucinations, seizures, encephalitis, such as limbic encephalitis or autoimmune encephalitis, epilepsy, neuropsychiatric symptoms, neuromyelitis optica spectrum disorder (NMOSD), neuromyotonia, Multiple Sclerosis, peripheral polyneuropathy, Guillain-Barré syndrome (GBS) symptoms, tetraparesis, cerebellar/brainstem syndrome, hearing loss, symmetric maculopathy, cerebellopontine lesion extending into spinal cord, personality changes, cognitive impairment and affect disorder, ataxia, such as gait and stand ataxia, neuropathy and multiple cerebral ischemia, preferably from the group consisting of PNS, dementia, cognitive decline, encephalitis, such as limbic encephalitis or autoimmune encephalitis, ataxia, such as gait and stand ataxia, neuromyelitis optica spectrum disorder (NMOSD), neuromyotonia, neuropathy, Multiple Sclerosis, epilepsy and neuropsychiatric symptoms. In another preferred embodiment, the autoimmune disease is associated with a cancer such as carcinoma, cerebral metastasis, leukemia and melanoma.

In a preferred embodiment, the polypeptide is a recombinant, isolated and/or purified polypeptide.

In a preferred embodiment, the polypeptide comprising at least on epitope of ZSCAN5 comprises at least one amino acid sequence selected from the group comprising SEQ ID NO:40, SEQ ID NO:41 and SEQ ID NO:42 or a variant thereof. In one embodiment, the polypeptide comprising at least on epitope of ZSCAN5 comprises at least the amino acid sequence as set forth in SEQ ID NO:40 or a variant thereof. In one embodiment, the polypeptide comprising at least on epitope of ZSCAN5 comprises at least the amino acid sequence as set forth in SEQ ID NO:41 or a variant thereof. In one embodiment, the polypeptide comprising at least on epitope of ZSCAN5 comprises at least the amino acid sequence as set forth in SEQ ID NO:42 or a variant thereof.

In a preferred embodiment, the mammalian antibody is an autoantibody. In one embodiment, the antibody is a human antibody. In one embodiment, the sample is a mammalian, preferably human sample comprising a representative set of antibodies. In a preferred embodiment, the sample is selected from the group comprising whole blood, plasma, serum, cerebrospinal fluid and saliva. In one embodiment, the sample is a sample previously obtained from a patient. In a preferred embodiment, the mammalian antibody comprises a human Fc region, such as described herein, optionally in combination with a binding region derived from a non-human species. In one embodiment, the mammalian antibody is isolated and/or purified. In one embodiment, the mammalian antibody is in a blood sample, wherein the blood sample is optionally diluted.

In a preferred embodiment, the mammalian antibody or complex is detected using a detection method selected from the group consisting of immunodiffusion, electrophoresis, light scattering immunoassays, agglutination, labeled immunoassays such as those from the group comprising radiolabeled immunoassay, enzyme immunoassays, more preferably ELISA, chemiluminescence immunoassays, preferably electrochemiluminescence immunoassay, and immunofluorescence, preferably indirect immunofluorescence.

In a preferred embodiment, the carrier is selected from the group consisting of a glass slide, preferably for microscopy, a biochip, a microtiter plate, a lateral flow device, a test strip, a membrane, preferably a line blot, a chromatography column and a bead, preferably a magnetic or fluorescent bead.

In one embodiment, the method or use of the present invention comprises contacting the carrier of the present invention with a means for detecting a mammalian antibody.

ZSCAN5 is a transcription factor occupying mammalian Pol III promoters and extra-TFIIIC (ETC) sites. Pol III transcripts are known to be essential to cellular survival. ZSCAN5 is in mammals represented by ZSCAN5B and in higher primates further comprises the paralogs ZSCAN5A, ZSCAN5C and ZSCAN5D (Sun et al. (2016), Oncotarget 7(45): 72571-72592).

SEQ ID NO:5 represents the amino acid sequence of ZSCAN5 as represented by ZSCAN5A of *Homo sapiens.* In one embodiment, ZSCAN5 comprises one or more, preferably all amino acid sequences selected from SEQ ID NO:1, SEQ ID NO:2, SEQ ID NO:3 and SEQ ID NO:4 and/or SEQ ID NO:9, SEQ ID NO:10, SEQ ID NO:11, SEQ ID NO:12, SEQ ID NO:13, SEQ ID NO:14, SEQ ID NO:15, SEQ ID NO:16 and SEQ ID NO:17 and/or SEQ ID NO:21, SEQ ID NO:22, SEQ ID NO:23 and SEQ ID NO:24 and/or SEQ ID NO:8 and/or SEQ ID NO:40.

SEQ ID NO:6 represents the amino acid sequence of ZSCAN5 as represented by ZSCAN5B of *Homo sapiens.* In one embodiment, ZSCAN5 comprises one or more, preferably all amino acid sequences selected from SEQ ID NO:1, SEQ ID NO:2, SEQ ID NO:3 and SEQ ID NO:4 and/or SEQ ID NO:18, SEQ ID NO:19 and SEQ ID NO:20 and/or SEQ ID NO:21, SEQ ID NO:22, SEQ ID NO:23 and SEQ ID NO:24 and/or SEQ ID NO:8 and/or SEQ ID NO:41.

SEQ ID NO:7 represents the amino acid sequence of ZSCAN5 as represented by ZSCAN5C of *Homo sapiens.* In one embodiment, ZSCAN5 comprises one or more, preferably all amino acid sequences selected from SEQ ID NO:1, SEQ ID NO:2, SEQ ID NO:3 and SEQ ID NO:4 and/or SEQ ID NO:9, SEQ ID NO:10, SEQ ID NO:11, SEQ ID NO:12, SEQ ID NO:13, SEQ ID NO:14, SEQ ID NO:15, SEQ ID NO:16 and SEQ ID NO:17 and/or SEQ ID NO:18, SEQ ID NO:19 and SEQ ID NO:20 and/or SEQ ID NO:8 and/or SEQ ID NO:42.

In a preferred embodiment, ZSCAN5 is from a mammal selected from the group consisting of a human, a non-human primate, a rodent, a cow, a horse, a dog, a cat, a bear, a donkey, a sheep, a goat, a camel and a dromedary, more preferably a human.

In a preferred embodiment, a method or use according to the present invention comprises the step providing the carrier according to the present invention. At least one polypeptide comprising at least one epitope of ZSCAN5 or a variant thereof may be immobilized on a solid surface of the carrier, or a means for capturing an antibody and/or a means for detecting an antibody may be configured for immobilizing the at least one polypeptide of the invention on the carrier, which may be done at a later stage, but before removing the sample and washing the carrier. The carrier or the polypeptide, if it is not already immobilized on the carrier, may then be contacted with the sample suspected of comprising the antibody under conditions allowing for binding of any antibodies to ZSCAN5. The polypeptide, if it is not already immobilized on the carrier, may then be immobilized on the carrier. However, the skilled person will readily understand that this can also be done during a later step of the method or use, if required at all. The sample may then be removed and the carrier or polypeptide may be washed to remove any remaining sample. A means for detecting the antibody carrying a detectable label such as a fluorescent dye may then be contacted with the carrier or polypeptide under conditions allowing formation of a complex between any bound antibody and the means. The carrier or polypeptide may be washed again. Finally, the presence of the antibody may be detected by checking whether the means such as a secondary antibody may be detected. Preferably an ELISA or an immunofluorescence assay using a mammalian cell or tissue expressing a polypeptide comprising the antigen of the invention or a variant thereof may be used. In the case of immunofluorescence, a distinct pattern determined by the expression pattern of the antigen in the cell or the tissue may indicate the presence of the antibody, as carried out or shown in the examples. In the case of an ELISA or another semi-quantitative or quantitative detection method a value above a cut off value determined as known in the art may indicate the presence of the antibody.

In one embodiment, the method, use, antibody, carrier, or kit described herein may be used for providing data, such as data regarding the presence or absence of an antibody in a sample or liquid, e.g., that may allow a clinician to conclude on part of or a clinical picture, together with other factors. To avoid any doubt, providing data, e.g. regarding the presence or absence of an antibody to ZSCAN5 does not allow to reach a diagnosis, taken alone. It may, however, help the clinician to direct their endeavor to reach a diagnosis in a certain direction.

A competitive assay, a capture bridge assay, an immunometric assay, a class-specific second antibody on the solid phase, a direct or indirect class capture assay may also be used. The principle of each of these formats is detailed in The Immunoassay Handbook, 3rd edition, edited by David Wild, Elsevier, 2005. Briefly, in a competitive format, the antibody to be detected may compete with a recombinant antibody for binding sites on the antigen, such as ZSCAN5 or at least one polypeptide comprising at least one epitope of ZSCAN5. Alternatively, a sample comprising the antibody may be preincubated with the antigen, followed by exposure to immobilized antigen or antigen configured for immobilization in one reaction and may be exposed to the antigen without pre-incubation with the antigen in another reaction to show specific binding. In a capture bridge assay, two antigen molecules bind to two antigen binding sites on the antibody to be detected. One of the antigen molecules is labeled and the other one immobilized or configured for immobilization, preferably via an affinity tag and a ligand binding specifically to said affinity tag. In an immunometric assay, the antibody to be detected binds to the antigen, which may be immobilized after or before the binding. The antibody may be detected using a means for detecting an antibody such as a secondary antibody. In a direct class capture assay, the antibody to be detected may be immobilized using a means for capturing an antibody and detected using a labeled antigen. In an indirect class capture assay, the antibody to be detected may be immobilized using a means for capturing an antibody. It may be detected using at least one, preferably two molecules of the antigen which bind to the antibody to be detected, and a means for detecting an antibody such as a secondary antibody. A secondary antibody may optionally be labeled.

In a preferred embodiment, the carrier and a means for capturing and/or a means for detecting a mammalian antibody are configured for immobilizing the means on the solid surface of the carrier. In another preferred embodiment, the carrier and the polypeptide of the invention are configured for immobilizing the polypeptide and/or the polypeptide is immobilized on a solid such as the carrier. A particularly preferred way for the configuration or the immobilization is modifying the carrier and/or the means or the carrier and/or the polypeptide, respectively, such that they comprise an affinity tag and a ligand to the affinity tag. Alternatively, the carrier or the means or the polypeptide, respectively, may comprise reactive chemical groups such as thiol, amino, epoxide, ester and anhydride groups. In a preferred embodiment, the term "immobilized", as used herein, refers to a molecule bound to a solid carrier insoluble in an aqueous solution, more preferably via a covalent or non-covalent bond, electrostatic interactions, encapsulation, unspecific absorption, printing or entrapment, for example by denaturing a globular polypeptide in a gel, or via hydrophobic interactions, most preferably via one or more covalent bonds. Various suitable carriers, for example paper, polystyrene, metal, silicon or glass surfaces, microfluidic channels, membranes, beads such as magnetic beads, column chromatography media, biochips, polyacrylamide gels and the like have been described in the literature, for example in Kim, D., and Herr, A. E. (2013), Protein immobilization techniques for microfluidic assays, Biomicrofluidics 7(4), 041501. This way, the immobilized molecule, together with the insoluble carrier, may be separated from an aqueous solution in a straightforward manner, for example by centrifugation or decanting. An immobilized molecule may be immobilized in a reversible or irreversible manner. For example, the immobilization is reversible if the molecule interacts with the carrier via ionic interactions that can be masked by addition of a high concentration of salt or if the molecule is bound via a cleavable covalent bond such as a disulfide bridge which may be cleaved by addition of thiol-containing reagents. By contrast, the immobilization is irreversible if the molecule is tethered to the carrier via a covalent bond that cannot be cleaved in aqueous solution, for example a bond formed by reaction of an epoxide group and an amine group as frequently used to couple lysine side chains to affinity columns. The protein may be indirectly immobilized, for example by immobilizing an antibody or other entity having affinity to the molecule, followed by formation of a complex to the effect that the molecule-antibody complex is immobilized. Various ways to immobilize molecules are described in the literature, for example in Kim, D., and Herr, A. E. (2013), Protein immobilization techniques for microfluidic assays, Biomicrofluidics 7(4), 041501. In addition, various reagents and kits for immobilization reactions are commercially available, for example from Pierce Biotechnology.

According to the present invention, a cell may be provided, which comprises an expression vector comprising a nucleotide sequence encoding at least one polypeptide comprising at least one epitope of ZSCAN5 or a variant thereof under the control of a promotor, optionally a strong and/or inducible promotor. The vector may encode for an N-terminal or C-terminal affinity tag fused to the at least one polypeptide, preferably via a linker sequence. The cell may be cultured under conditions allowing for the expression of the at least one polypeptide of the invention. Any expressed polypeptide may then be purified, preferably using the affinity tag. However, a non-purified polypeptide may also be used in some embodiments. Subsequently it may be immobilized on the carrier according to the present invention. In a preferred embodiment, the cell, nucleic acid or vector may be used for the manufacture of a kit, optionally for the diagnosis or aiding in the diagnosis of an autoimmune disease, such as a neurological autoimmune disease.

According to the present invention, a medical or diagnostic device such as the carrier, preferably diagnostically useful carrier, of the invention may be prepared by expressing a recombinant polypeptide comprising a variant of ZSCAN5 or of at least one epitope of ZSCAN5 or a variant thereof comprising an affinity tag, optionally with an artificial linker, which may include a protease cleavage site, in a cell such as a eukaryotic or prokaryotic cell. Contacting the polypeptide with a ligand binding specifically to the affinity tag, which ligand may be immobilized on a solid phase, washing the solid phase such that non-specifically bound material from the cell is removed and eluting the expressed variant from the solid phase, preferably by adding an excess of non-immobilized ligand. The variant may then be immobilized on the device. Optionally, the affinity tag may be removed by contacting the variant with a protease, preferably a protease recognizing the protease cleavage site, before the immobilization. The affinity tag may be selected from the group of tags comprising His, immobilized nickel, glutathione, chitin, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofΓag, Streptavidin, Strep-tag II, T7 Epitope Tag, TAP, TC, Thioredoxin, Ty, V5, VSV, biotin, Xpress Tag and a recombinant antibody binding to the ligand to an affinity tag. Useful proteases include, but are not limited to TEV, Thrombin, Faktor Xa or Enteropeptidase. Suitable linkers are part of vectors, for example pET vector series (Novagen).

In a preferred embodiment, the absence or presence of two or more mammalian antibodies to two or more polypeptides each comprising at least one epitope of ZSCAN5 is detected simultaneously, *i.e.* at the same time.

In another preferred embodiment, the presence or absence of at least one mammalian antibody other than a mammalian antibody binding specifically to ZSCAN5 is detected, preferably from the group consisting of a mammalian antibody binding specifically to NMDAR, a mammalian antibody binding specifically to Lgl1, a mammalian antibody binding specifically to AMPA1, a mammalian antibody binding specifically to AMPA2, a mammalian antibody binding specifically to CASPR2, a mammalian antibody binding specifically to GABA B, a mammalian antibody binding specifically to GABA A, a mammalian antibody binding specifically to DPPX, a mammalian antibody binding specifically to IGLON5, a mammalian antibody binding specifically to Hu, a mammalian antibody binding specifically to Yo, a mammalian antibody binding specifically to CRMP5, a mammalian antibody binding specifically to Ri, a mammalian antibody binding specifically to Ma2, a mammalian antibody binding specifically to Amphiphysin, a mammalian antibody binding specifically to Recoverin, a mammalian antibody binding specifically to RGS8, a mammalian antibody binding specifically to DAGLA, a mammalian antibody binding specifically to NSF, a mammalian antibody binding specifically to STX1B, a mammalian antibody binding specifically to DNM1, a mammalian antibody binding specifically to VAMP2, a mammalian antibody binding specifically to Anna-3, a mammalian antibody binding specifically to Zic-4, a mammalian antibody binding specifically to SOX1 (US7314721), a mammalian antibody binding specifically to PCA2, a mammalian antibody binding specifically to Tr, a mammalian antibody binding specifically to glutamic acid decarboxylase, a mammalian antibody binding specifically to AK5, a mammalian antibody binding specifically to AP3B2, a mammalian antibody binding specifically to Flotillin1/2, a mammalian antibody binding specifically to GRM1, a mammalian antibody binding specifically to GRM2, a mammalian antibody binding specifically to GRM5, a mammalian antibody binding specifically to GLURD2, a mammalian antibody binding specifically to ITPR1, a mammalian antibody binding specifically to KCNA2, a mammalian antibody binding specifically to NCDN, a mammalian antibody binding specifically to Septin 3+5+6+7+11 and a mammalian antibody binding specifically to Sez6L2. In a preferred embodiment, any of these mammalian antibodies is a mammalian autoantibody, preferably human autoantibody. In a preferred embodiment, the detection of the presence of any of these mammalian antibodies aids in the diagnosis or implies a diagnosis of an autoimmune disease, such as a neurological autoimmune disease. In a preferred embodiment, two or more mammalian antibodies are detected in the same sample and preferably essentially simultaneously. The carrier according to the present invention may be configured for detecting two or more mammalian antibodies in the same sample, preferably essentially simultaneously.

In a preferred embodiment, the presence or absence of two or more mammalian antibodies is detected and can be distinguished. In other words, a signal indicating the presence of an antibody indicates which of the two antibodies is present. In a preferred embodiment, the absence or presence of two or more mammalian antibodies is detected in spatially separate reactions, more preferably in different reaction mixtures in separate vessels. In another preferred embodiment, a signal indicating the presence of one of the two antibodies may be distinguished from a signal indicating the presence of the other antibody. This may be achieved by using different detectable labels, more preferably distinguishable fluorophores. For example, a fluorophore emitting green light and another one emitting red light may be used.

In a preferred embodiment, the presence or absence of two or more mammalian antibodies is detected, but cannot be distinguished. In a preferred embodiment, their absence or presence is detected in a one pot reaction, preferably in two or more reactions in the same reaction vessel without spatial separation, and with no signal distinction. In other words, a signal indicates that at least one of the two or more antibodies is present, but not which one. In a preferred embodiment, two or more antigens, e.g. represented by two or more polypeptides each comprising at least one epitope of ZSCAN5, may be present in a mixture.

In a preferred embodiment, the sample comprises a representative set of antibodies, preferably mammalian antibodies, more preferably IgG, IgA and IgM class antibodies, most preferably IgG class antibodies. It is preferably selected from the group consisting of, optionally diluted, whole blood, plasma, serum, cerebrospinal fluid (CSF) and saliva. The sample may be a liquid sample or a dried blood spot made using the sample, preferably whole blood, plasma, serum or capillary blood, preferably capillary blood.

In a preferred embodiment, the sample is from an organism having a brain and producing antibodies, preferably from the group comprising mammals and birds, more preferably a mammal from the group consisting of a human, a non-human primate, a rodent (such as a rat or mouse), a cow, a horse, a dog, a cat, a bear, a donkey, a sheep, a goat, a camel and a dromedary, most preferably a human. In another preferred embodiment it is from a bird, more preferably from the group comprising a chicken, a parrot and a falcon.

The mammalian antibody to be detected or a means for detecting or capturing an antibody, such as a mammalian and/or immobilized antibody, binds specifically to its interaction partner, which may be an antigen, such as ZSCAN5 or a variant thereof, in the case of a mammalian antibody or an antibody in the case of a means for detecting an antibody, preferably the mammalian antibody binding specifically to ZSCAN5 or a variant thereof. In a preferred embodiment, the terms "specific binding", "binding specifically", "specifically capturing" and similar terms, as used herein, mean that the binding reaction is stronger than a binding reaction characterized by a dissociation constant of 1 × 10⁻⁵ M, more preferably 1 × 10⁻⁷ M, more preferably 1 × 10⁻⁸ M, more preferably 1 × 10⁻⁹ M, more preferably 1 × 10⁻¹⁰ M, more preferably 1 × 10⁻¹¹ M, more preferably 1 × 10⁻¹² M, as determined by surface plasmon resonance using Biacore equipment at 25 °C in PBS buffer at pH 7.

In a preferred embodiment, the mammalian antibody is a mammalian autoantibody. In a preferred embodiment, the term "autoantibody", as used herein, refers to an antibody which binds specifically to a structure, preferably an antigen, such as ZSCAN5, from the organism which produces said antibody, wherein the level of such antibody is preferably elevated compared to the level in the average healthy subject. Thus, an autoantibody is an endogenous molecule produced within a subject's body. The organism is preferably a patient suspected of or actually suffering from a disease, preferably a mammalian, more preferably human patient. Such an autoantibody has a constant region, as have other antibodies of the same class from the same organism. Typically, the constant region of a mammalian antibody such as an autoantibody may be composed of a heavy chain constant region (C_{H}) and a light chain constant region (C_{L}). In one embodiment, the mammalian (auto)antibody comprises a C_{H} selected from the group comprising SEQ ID NO:43, SEQ ID NO:44, SEQ ID NO:45, SEQ ID NO:46, SEQ ID NO:47, SEQ ID NO:48, SEQ ID NO:49, SEQ ID NO:50 and SEQ ID NO:51 or a variant thereof, preferably selected from the group comprising SEQ ID NO:47, SEQ ID NO:48, SEQ ID NO:49 and SEQ ID NO:50 or a variant thereof. In one embodiment, the mammalian (auto)antibody comprises a C_{L} selected from the group comprising SEQ ID NO:52, SEQ ID NO:53, SEQ ID NO:54, SEQ ID NO:55, SEQ ID NO:56 and SEQ ID NO:57 or a variant thereof. Particularly preferably, the autoantibody is a human autoantibody, more preferably a human autoantibody of class IgG, IgM or IgA, preferably IgG. The variable domain is capable of binding specifically against the antigen, such as ZSCAN5 or a variant thereof. In one embodiment, the constant region binds specifically to molecules recognizing the constant region of antibodies, such as IgG class antibodies, such as secondary antibodies. It may have sequence elements shared by other antibodies, such as IgG class antibodies, from the same organism. Thus, the autoantibody may have the sequence of an antibody's constant regions from the animal, preferably human, making it, but the variable region is able to bind specifically to the endogenous molecule of the animal, such as ZSCAN5 or a variant thereof. In a preferred embodiment, the (auto)antibody is isolated and/or purified from a sample, preferably tissue, serum, plasma, blood or CSF from the animal, preferably human. The autoantibody can be isolated as a mixture of polyclonal, native antibodies from the animal or patient. It is not a synthetic, monoclonal or recombinant antibody. A recombinant antibody binding specifically to ZSCAN5 or a variant thereof may be generated using standard methods known in the field. It may be useful as a positive control and for detection assays, for example sandwich assays or competitive assays.

According to the present invention, a mammalian antibody binding specifically to ZSCAN5 or a variant thereof is provided or isolated. The person skilled in the art is familiar with the isolation or purification of antibodies. Comprehensive instructions are available in the prior art, for example in Affinity Chromatography Vol. 1 Antibody by GE Healthcare, www. gel ifesciences. com, April 2016 and Thermo Scientific Pierce Antibody Production and Purification Technical Handbook, Version 2, www.thermoscientific.com. For example, specific purification steps may involve affinity chromatography using a polypeptide comprising the antigen of the invention or a variant thereof, such as at least one polypeptide comprising at least one epitope of ZSCAN5 or a variant thereof, immobilized to beads by coupling using primary amines and/or Protein G.

The teachings of the present invention may not only be carried out using the polypeptides, in particular at least one polypeptide comprising the native sequence of at least one epitope of ZSCAN5 or nucleic acids having the exact sequences referred to in this application explicitly, for example by function, name, sequence or accession number, or implicitly, but also using variants of such polypeptides or nucleic acids. For the avoidance of doubt, in preferred embodiments, a polypeptide comprising at least one epitope of ZSCAN5 comprises a polypeptide comprising the entire amino acid sequence of ZSCAN5 or a variant thereof which is not truncated at one or both termini of ZSCAN5. However, in other preferred embodiments, the term also relates to a polypeptide, which does not comprise the entire amino acid sequence of ZSCAN5, e.g., which comprises an amino acid sequence of ZSCAN5 which is truncated at one or both termini, as long as it comprises at least one epitope of ZSCAN5, preferably immunogenic epitope, or variant thereof, as described herein.

In a preferred embodiment, the term "variant", as used herein, may refer to at least one fragment of the full-length sequence referred to, more specifically one or more amino acid or nucleic acid sequence which is, relative to the full-length sequence, truncated at one or both termini by one or more amino acids or nucleic acids, respectively. Such a fragment comprises or encodes for a peptide having at least 6, 7, 8, 9, 10, 12, 15, 16, 17, 18, 19, 20, 25, 30, 35, 40, 50, 75, 100, 150, 200, 250, 300, 350, 400, 410, 420, 430, 440, 450, 460, 470, 480 or 490 successive amino acids of the original sequence or a variant thereof. The total length of the variant may be at least 6, 7, 8, 9, 10, 12, 15, 16, 17, 18, 19, 20, 25, 30, 35, 40 50, 75, 100, 150, 200, 250, 300, 350, 400, 410, 420, 430, 440, 450, 460, 470, 480 or 490 or more amino acids.

The term "variant" relates not only to at least one fragment, but also to a polypeptide or a fragment thereof comprising amino acid sequences that are at least 40, 50, 60, 70, 75, 80, 85, 90, 92, 94, 95, 96, 97, 98, 99, 99,3, 99,4, 99,5, 99,6, 99,7, 99,8 or 99,9, preferably at least 80% or 99% identical to the reference amino acid sequence referred to or the fragment thereof, wherein amino acids other than those essential for the biological activity, for example the ability of an antigen to bind to an antibody, or the fold or structure of the polypeptide are deleted or substituted and/or one or more such essential amino acids are replaced in a conservative manner and/or amino acids are added such that the biological activity of the polypeptide is preserved. In other words, in preferred embodiments the variant maintains the biological activity (of the full-length protein of which it is derived). The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948) is used using default setting.

In a preferred embodiment, the polypeptide and variants thereof may comprise a tag, such as an affinity tag, wherein the tag preferably is selected from the group comprising His tag, GST tag, E tag, FLAG tag, HA tag, myc tag, V5 tag, S tag, SnoopTag, SpyTag, SofTag, Strep tag, Strep tag II, T7 Epitope tag, immobilized nickel, glutathione, chitin, 18A, biotin tag, ALFA-tag, AviTag, ACP, BCCP, Calmodulin, Chitin binding protein, ELK16, C-Tag, calmodulin tag, iCap tag, polyglutamate tag, polyarginine tag, NE-tag, Rho1D4-tag, SBP-tag, Softag 1, Softag 3, Spot-tag, T7-tag, TC tag, Ty tag, VSV-tag, TAP tag, thioredoxin, flash, poly aspartate, Isope, maltose binding protein, myc, nus, NE, ProtA, ProtC, Tho1d4 and Xpress tag.

In a preferred embodiment, the polypeptide and variants thereof may, in addition, comprise chemical modifications, for example isotopic labels or covalent modifications such as glycosylation, phosphorylation, acetylation, decarboxylation, citrullination, methylation, hydroxylation, ubiquitylation and the like. The person skilled in the art is familiar with methods to modify polypeptides. Any modification is designed such that it does not abolish the biological activity of ZSCAN5 or epitope thereof or variant of the protein or epitope thereof.

Moreover, variants may also be generated by N- or/and C-terminal fusion of polypeptides, fragments or variants thereof with other known polypeptides or variants thereof or artificial sequences such as linkers and comprise active portions or domains, preferably having a sequence identity of at least 70, 75, 80, 85, 90, 92, 94, 95, 96, 97, 98 or 99% when aligned with the active portion of the reference sequence, wherein the term "active portion", as used herein, refers to an amino acid sequence, which is less than the full length amino acid sequence or, in the case of a nucleic acid sequence, codes for less than the full length amino acid sequence, respectively, and/or is a variant of the natural sequence, but retains at least some of the biological activity. Preferably the active portion is an active portion of ZSCAN5. The polypeptide may comprise additional sequences, preferably artificial sequences for example linkers or binding epitopes. Any fused sequences are chosen such that the ability of the antigen of the invention or a variant thereof to bind specifically to the antibody to be detected or the diagnostic reliability, in particular sensitivity and/or specificity, is not significantly altered, let alone abolished.

In a preferred embodiment, the term "variant" of a nucleic acid comprises nucleic acids the complementary strand of which hybridizes, preferably under stringent conditions, to the reference or wild type nucleic acid. Stringency of hybridization reactions is readily determinable by one of ordinary skill in the art, and in general is an empirical calculation dependent on probe length, washing temperature and salt concentration. For additional details and explanation of stringency of hybridization reactions, see Ausubel, F. M. (1995), Current Protocols in Molecular Biology. John Wiley & Sons, Inc. Moreover, the person skilled in the art may follow the instructions given in the manual Boehringer Mannheim GmbH (1993) The DIG System Users Guide for Filter Hybridization, Boehringer Mannheim GmbH, Mannheim, Germany and in Liebl, W., Ehrmann, M., Ludwig, W., and Schleifer, K. H. (1991) International Journal of Systematic Bacteriology 41: 255-260 on how to identify DNA sequences by means of hybridization. In a preferred embodiment, the term variant of a nucleic acid sequence, as used herein, refers to any nucleic acid sequence that encodes the same amino acid sequence, preferably at least one polypeptide comprising at least one epitope of ZSCAN5 or a variant thereof, as the reference nucleic acid sequence, in line with the degeneracy of the genetic code.

In a preferred embodiment, the term "sensitivity" refers to the number of samples correctly determined as positive relative to the total number of samples examined. In a preferred embodiment, the term "specificity" refers to the number of samples correctly determined as negative relative to the total number of samples examined.

The variant of an antigen described herein, such as ZSCAN5, or at least one polypeptide comprising at least one epitope of an antigen described herein, such as ZSCAN5, has biological activity. In a preferred embodiment, such biological activity is the ability to bind specifically to a mammalian antibody binding specifically to the antigen, such as ZSCAN5, e.g., as found in a patient suffering from an autoimmune disease, such as a neurological autoimmune disease, preferably associated with the presence of such antibody in a sample. In the case of ZSCAN5, the autoimmune disease, such as the neurological autoimmune disease, is preferably selected from the group consisting of PNS, dementia, cognitive decline, psychotic symptoms, acoustic hallucinations, seizures, encephalitis, such as limbic encephalitis or autoimmune encephalitis, epilepsy, neuropsychiatric symptoms, NMOSD, neuromyotonia, neuropathy, Multiple Sclerosis, peripheral polyneuropathy, Guillain-Barré syndrome (GBS) symptoms, tetraparesis, cerebellar/brainstem syndrome, hearing loss, symmetric maculopathy, cerebellopontine lesion extending into spinal cord, personality changes, cognitive impairment and affect disorder, ataxia, such as gait and stand ataxia, multiple cerebral ischemia, and/or cancers such as carcinoma, cerebral metastases and melanoma. In preferred embodiments, the dementia is a rapidly progressive dementia. In other preferred embodiments, the encephalitis is limbic encephalitis or autoimmune encephalitis, optionally in combination with epilepsy and/or neuropsychiatric symptoms. In further preferred embodiments, the carcinoma is a mamma carcinoma or a lung carcinoma (NSCLC). For example, whether or not a variant of the polypeptide has such biological activity may be checked by determining whether or not it binds specifically to a mammalian antibody from a sample of such a patient comprising a mammalian antibody binding specifically to the wild type antigen, such as ZSCAN5, preferably as determined by indirect immunofluorescence, e.g. as described in the experimental section of this application. In a preferred embodiment, the person skilled in the art will, when designing variants consider that domains exposed to the cytoplasm or extracellular space are likely to contain epitopes of the mammalian antibody to be detected. In even more preferred embodiments, the variant of ZSCAN5 may be a polypeptide comprising at least one sequence selected from the group comprising SEQ ID NO:1, SEQ ID NO:2, SEQ ID NO:3 and SEQ ID NO:4 or a variant thereof and/or SEQ ID NO:8 or a variant thereof and/or from the group comprising SEQ ID NO:9, SEQ ID NO:10, SEQ ID NO:11, SEQ ID NO:12, SEQ ID NO:13, SEQ ID NO:14, SEQ ID NO:15, SEQ ID NO:16 and SEQ ID NO:17 or a variant thereof and/or from the group comprising SEQ ID NO:18, SEQ ID NO:19 and SEQ ID NO:20 or a variant thereof and/or from the group comprising SEQ ID NO:21, SEQ ID NO:22, SEQ ID NO:23 and SEQ ID NO:24 or a variant thereof and/or from the group comprising SEQ ID NO:40, SEQ ID NO:41 and SEQ ID NO:42 or a variant thereof. In an even more preferred embodiment, the variant is a polypeptide comprising at least SEQ ID NO:8 or a variant thereof. In another even more preferred embodiment, the variant is a polypeptide comprising at least one amino acid sequence selected from the group comprising SEQ ID NO:40, SEQ ID NO:41 and SEQ ID NO:42 or a variant thereof.

The polypeptide used to carry out the inventive teachings, including any variants, is preferably designed such that it comprises at least one epitope recognized by and/or binding specifically to an antibody binding specifically to an antigen, such as ZSCAN5. Said epitope may demonstrate strongest binding to an antibody binding to the antigen, such as ZSCAN5, compared with the binding observed towards other antibodies. In one embodiment, such polypeptide comprises a stretch of 6, 7, 8, 9, 10, 11, 12, 20, 25, 30, 40, 50, 60, 70, 80, 90, 100, 113, 125 or more, preferably at least 9 but no more than 16, consecutive amino acids from the antigen, such as ZSCAN5, as described herein. The person skilled in the art is familiar with guidelines used to design peptides having sufficient immunogenicity, for example those described in Jackson, D. C., Fitzmaurice, C. J., Brown, L. E., Zeng, W. (1999), Preparation and properties of totally synthetic immunogenes, Vaccine Volume 18, Issues 3-4, September 1999, Pages 355-361; and Black, M., Trent, A., Tirrell, M. and Olive, C. (2010), Advances in the design and delivery of peptide subunit vaccines with a focus on Toll-like receptor agonists, Expert Rev Vaccines, 2010 February; 9(2): 157-173. Briefly, it is desirable that the peptide meets as many as possible of the following requirements: (a) it has a high degree of hydrophilicity, (b) it comprises one or more residues selected from the group comprising aspartate, proline, tyrosine and phenylalanine, (c) is has, for higher specificity, no or little homology with other known peptides or polypeptides, (d) it needs to be sufficiently soluble and (e) it comprises no glycosylation or phosphorylation sites unless required for specific reasons. Alternatively, bioinformatics approaches may be followed, for example those described by Moreau, V., Fleury, C., Piquer, D., Nguyen, C., Novali, N., Villard, S., Laune, D., Granier, C. and Molina, F. (2008), PEPOP: Computational design of immunogenic peptides, BMC Bioinformatics 2008, 9:71.

The inventive polypeptide, when used according to the present invention, may be provided in any kind of conformation. For example, the polypeptide may be an essentially unfolded, a partially or a fully folded polypeptide. In a preferred embodiment, the polypeptide is folded in the sense that the epitope(s) essential for the binding to the inventive antibody, or the protein or variant thereof in its entirety, adopt the fold adopted by the native protein in its natural environment. The person skilled in the art is familiar with methods suitable to determine whether or not a polypeptide is folded and if it is, which structure it has, for example limited proteolysis, NMR spectroscopy, CD spectroscopy or X-ray crystallography (see for example Banaszak L. J. (2008), Foundations of Structural Biology, Academics Press, or Teng Q. (2013), Structural Biology: Practical Applications, Springer), preferably CD spectroscopy is used.

The inventive polypeptide may be a fusion protein which comprises amino acid sequences other than those taken from ZSCAN5, in particular a C-terminal or N-terminal tag, preferably a N-terminal tag, which is, in a preferred embodiment, an additional sequence motif or polypeptide having a function that has some biological or physical function and may, for example, be used to purify, immobilize, precipitate or identify the inventive polypeptide. In a more preferred embodiment, the tag is a sequence or domain capable of binding specifically to a ligand, for example a tag selected from the group comprising His tags, thioredoxin, maltose binding protein, glutathione-S-transferase, a fluorescence tag, for example from the group comprising green fluorescent protein.

According to the present invention, at least one cell is provided which overexpresses at least one polypeptide comprising at least one epitope of ZSCAN5 or a variant thereof, preferably in combination with at least one additional cell which overexpresses another antigen from the group consisting of Hu, Yo, Ri, CV2, PNMA1, PNMA2, DNER/Tr, ARHGAP26, ITPR1 (EP14003703.7), ATP1A3, NBC1 (EP14003958.7), Neurochrondrin (EP15001186), CARPVIII, Zic4, SOX1 (US7314721), Ma, MAG, MP0, MBP, GAD65, amphiphysin, recoverin, GABA A receptor, GABA B receptor, glycine receptor, gephyrin, IgLON5, DPPX, aquaporin-4, MOG, NMDA receptor, AMPA receptors, GRM1, GRM5, LGI1, VGCC, mGluR1, CASPR2, ATP1A3, also referred to as alpha 3 subunit of human neuronal Na(+)/K(+) ATPase (EP14171561.5) and Flotillin1/2 (EP3101424) a variant thereof comprised in a polypeptide.

In a preferred embodiment, the term "overexpressing", as used herein, means that the cell has been transfected with a nucleic acid that comprises a nucleic acid sequence encoding at least one polypeptide comprising at least one epitope of ZSCAN5 or a variant thereof, or the other antigen described herein, or a variant thereof under the control of a promotor. Consequently, the transfected cell expresses more polypeptide recognized by the mammalian antibody binding specifically to be detected than the same type of cell normally would, probably at least 10, 20, 30, 50, 100, 200 or 500% more as judged by quantitative Western Blot. The promotor may be an inducible promotor, which allows for the induction of expression by addition of an inducer. The person skilled in the art is familiar with protocols and vectors for transiently overexpressing a polypeptide in a eukaryotic cell, for example the pTriEx system from Novagen and with protocols and vectors for stably transfecting a eukaryotic cell, for example the pcDNA^{™}4/TO vector system from Invitrogen.

In a preferred embodiment, a fixed mammalian cell may be used. In a preferred embodiment, the term "fixed" cell, as used herein, refers to a cell that has been treated with a reactive chemical compound to the effect that the cell is no longer metabolically active, but still presents its epitopes for immunostaining with antibodies and their subsequent detection, for example by fluorescence. More preferably, the reactive chemical compound is selected from the group comprising acetone, formalin, methanol and ethanol or mixtures thereof, preferably all of them. The person skilled in the art is familiar with protocols that may be used to prepare fixed cells.

In one embodiment, the cell may be on a carrier for microscopic immunofluorescence analysis, which may further comprise a mock transfected cell which may serve as a negative control. Such a carrier may be a glass slide. The cell on the carrier such as the glass slide may be covered with a mounting buffer. A mounting buffer is a liquid which helps maintain a near physiological pH to maintain the molecular structure of any diagnostically relevant molecule and their epitopes, is compatible with the emission of a fluorescence signal and prevents a premature loss of fluorescence due to bleaching of the fluorophore. It may be selected from the group consisting of water, glycerol, natural oil or plastic or a mixture thereof, preferably water and glycerol. Various compositions are described in the state of the art, for example in "Mountants and Antifades", published by Wright Cell Imaging Facility, Toronto Western Research Institute University Health Network, (https://de.scribd.com/document/47879592/Mountants-Antifades), Krenek et al. (1989) J. Immunol. Meth 117, 91-97 and Nairn et al. (1969) Clin. Exp. Immunol. 4, 697-705. Preparation and evaluation of cells using microscopy is widely known in the field and described, e.g., in "IIFT: Neurology Mosaics Instructions for use", FA_112d-1_A_UK_C17.doc, V. 04/05/2022 from EUROIMMUN Medizinische Labordiagnostika, AG.

In a preferred embodiment, the cell is a eukaryotic cell overexpressing the polypeptide, such as a cell selected from the group comprising HEK, Hela, CHO and Jurkat cells and derivatives thereof. In a preferred embodiment, the cell is a recombinant cell overexpressing the polypeptide, which is preferably under the control of a heterologous strong promoter.

In another preferred embodiment, the carrier such as the diagnostically useful carrier, is a bead. Various beads for numerous applications are commercially available, mainly based on carbohydrate, for example sepharose or agarose, or plastic. They may contain active or activatable chemical groups such as a carboxyl or tosyl or ester group, which can be utilized for the immobilization of a means for specifically capturing an antibody. Preferably, the beads are beads having an average diameter of from 0.1 µm to 10 µm, from 0.5 µm to 8 µm, from 0.75 µm to 7 µm or from 1 µm to 6 µm. Preferably, the bead is provided in the form of an aqueous suspension having a bead content of from 10 to 90%, preferably from 20 to 80%, preferably from 30 to 70%, more preferably from 40 to 60% (w/w). The person skilled in the art is familiar with such beads (Diamindis, E. P., Chriopoulus, T. K., Immunoassays, 1996, Academic Press), which are commercially available, for example Bio-Plex COOH beads MC10026-01 or 171-506011 from Bio-Rad.

In another preferred embodiment, the carrier is a microtiter plate comprising a multitude of wells such as at least 8 wells that may be used for ELISA. At least one of the wells is coated with the means for specifically capturing an antibody, either directly or indirectly, preferably at least one polypeptide comprising at least one epitope of ZSCAN5 or a variant thereof. At least 3, preferably 4, more preferably 5, or even more calibrators, at defined concentrations may be used to set up a calibration curve for semi-quantitative analysis. When the inventive method is carried out, the calibrators, which typically cover a range of concentrations covering the calibrating curve, may be processed and developed in parallel to the samples. A secondary antibody comprising a detectable label such as an enzymatically active label may be provided, for example a label having horse radish peroxidase activity or alkaline phosphatase activity or an enzyme capable of chemiluminescence.

In another preferred embodiment, the carrier is a microarray. In a preferred embodiment, the term "microarray", as used herein, refers to a chip spotted with a variety of spatially separate antigens, preferably at least 5, more preferably 10, 20, 30, 40, 50, 80 or 100 or even more. Preferably each antigen is a peptide comprising or consisting of 5 to 25, preferably 7 to 16 successive amino acids spanning a fragment of a mammalian protein. At least one antigen is a polypeptide comprising at least one epitope of ZSCAN5 or a variant thereof. Preferably two or more antigens are polypeptides each comprising at least one epitope of ZSCAN5 or a variant thereof, as described herein. A secondary antibody comprising a label, preferably a fluorescent label, and specifically binding to anti-human IgG may be used for the detection. Preferably at least one additional antigen or a variant thereof is spotted.

In a preferred embodiment, the presence of a mammalian antibody binding to an antigen described herein, such as ZSCAN5, in a sample from a subject is determined using ELISA. In another preferred embodiment, the presence of a mammalian antibody binding to an antigen described herein, such as ZSCAN5, in a sample from a subject is determined using immunofluorescence, preferably indirect immunofluorescence.

According to the present invention, a means for detecting an antibody is used, which is a molecule binding specifically to the antibody and allowing detection, typically comprising a detectable label. In one embodiment, the means for detecting an antibody is a secondary antibody or a polypeptide comprising at least one epitope of an antigen, such as ZSCAN5, wherein the means binds to the antibody to be detected. In a preferred embodiment, a detectable label may be used to distinguish a population of molecules from others using biophysical detection methods. It is preferably selected from the group comprising a fluorescent, a radioactive, a chemiluminescent label, a heavy metal such as gold label, a nanoparticle, a bead or an enzymatically active label, preferably one catalyzing a colorimetric reaction. In a preferred embodiment, a fluorescent label is selected from the group comprising Alexa dyes, FITC, TRITC and green fluorescent protein (GFP). lodine-125 may be used as radioactive label. In a preferred embodiment, an enzymatically active label is selected from the group comprising horseradish peroxidase, glucose oxidase, beta galactosidase, alkaline phosphatase and luciferase. In a preferred embodiment, a chemiluminescent label is selected from the group comprising luminol or a derivative, an acridinium ester and luciferase. The person skilled in the art is able to choose suitable labels and to attach them to proteins, nucleic acids and other molecules (Hassanzadeh L, Chen S, Veedu RN. Radiolabeling of Nucleic Acid Aptamers for Highly Sensitive Disease-Specific Molecular Imaging. Pharmaceuticals (Basel). 2018;11(4):106. Published 2018 Oct 15. doi: 10.3390/ph11040106 Hassanzadeh L, Chen S, Veedu RN. Radiolabeling of Nucleic Acid Aptamers for Highly Sensitive Disease-Specific Molecular Imaging. Pharmaceuticals (Basel). 2018;11(4):106. Published 2018 Oct 15. doi:10.3390/ph11040106, Bioconjugate Techniques, 3rd Edition (2013) by Greg T. Hermanson, Obermaier C, Griebel A, Westermeier R. Principles of protein labeling techniques.

Methods Mol Biol. 2015; 1295: 153-65), and a wide range of labeled molecules are commercially available. According to the present invention, a means for capturing an antibody such as an IgG class antibody is a molecule binding specifically to the antibody to be immobilized and capable of immobilizing it, either because it is immobilized itself or configured for immobilization, preferably *via* an affinity tag. In one embodiment, the means for detecting an immobilized antibody may be selected from the group comprising a secondary antibody binding to a mammalian antibody, such as a secondary antibody binding to IgG, preferably IgG1 and/or IgA and/or IgM, a polypeptide comprising ZSCAN5 or a variant thereof, a polypeptide comprising at least one epitope of ZSCAN5 or a variant thereof, a ligand binding specifically to ZSCAN5, Protein G or a variant thereof or an aptamer or an antibody binding specifically to the immobilized antibody.

In accordance with the present invention, the term "secondary antibody" in its broadest sense is to be understood to refer to any kind of "binding moiety", preferably binding protein, capable of specific binding to an IgA, IgG and/or IgM class antibody or a fragment thereof such as a constant region of a particular Ig class of a selected species, preferably human species, such as described herein. Non-limiting examples of binding moieties include antibodies, for example antibodies immunologically or genetically derived from any species, for example human, chicken, camel, llama, lamprey, shark, goat, rodent, cow, dog, rabbit, etc., antibody fragments, domains or parts thereof, for example Fab, Fab', F(ab')₂, scFab, Fv, scFv, VH, VHH, VL, VLRs, and the like, diabodies, monoclonal antibodies (mAbs), polyclonal antibodies (pAbs), mAbdAbs, phage display-derived binders, affibodies, heteroconjugate antibodies, bispecific antibodies, evibodies, lipocalins, anticalins, affibodies, avimers, maxibodies, heat shock proteins such as GroEL and GroES, trans-bodies, DARPins, aptamers, C-type lectin domains such as tetranectins; human γ-crystallin and human ubiquitin-derived binders such as affilins, PDZ domain-derived binders; scorpion toxin and/or Kunitz-type domain binders, fibronectin-derived binders such as adnectins, receptors, ligands, lectins, streptavidin, biotin, including derivatives and/or combinations thereof such as bi-/multi-specific formats formed from two or more of these binding molecules. Various antibody-derived and alternative (i.e. non-antibody) binding protein scaffolds including methods of generation thereof are known in the art (e.g. reviewed in Chiu ML et al., Antibodies (Basel), (2019);8(4):55; Simeon R. & Chen Z., Protein Cell. (2018);9(1):3-14; and Chapter 7 - Non-Antibody Scaffolds from Handbook of Therapeutic Antibodies (2007) edited by Stefan Dübel, US 7,166,697, Rothe C & Skerra A., BioDrugs. (2018);32(3):233-243; Gebauer M & Skerra A, Curr Opin Biotechnol. (2019); 60:230-241; Feldwisch, J & Tolmachev, V. (2012) Methods Mol. Biol. 899:103-126; Wikman M etal., Protein Eng Des Sel. (2004);17(5):455-62; Silverman J et al. (2005), Nat Biotechnol 23:1556-1561; Plückthun A., Annu Rev Pharmacol Toxicol. (2015);55:489-511; Hosse RJ *et al.* (2006).

Protein Sci 15:14-27; Hackel BJ, et al. (2008) J Mol Biol 381:1238-1252. In a preferred embodiment, a secondary antibody is an antibody binding to all antibodies from an antibody or immunoglobulin class, preferably a human antibody class, preferably IgA and/or IgG and/or IgM antibodies, more preferably IgG. Secondary antibodies may recognize the constant region, such as described herein, of said class or one or more epitopes across the sequence or 3D structure shared by antibodies of the Ig class of interest. Secondary antibodies are typically from a mammal other than a human or from a bird, preferably from chicken, rabbit, mouse, rat, horse, pig, donkey, goat, cow, camel, llama, or non-human primate. A secondary antibody may be a monoclonal, preferably recombinant antibody or may be a polyclonal antibody. A wide range of them is commercially available. In preferred embodiments, the secondary antibody specifically binds to a human IgG, more preferably to the constant region of a human IgG, even more preferably to an amino acid sequence selected from the group comprising SEQ ID NO:47, SEQ ID NO:48, SEQ ID NO:49 and SEQ ID NO:50 or a variant thereof.

In a preferred embodiment, a ligand to an affinity tag, as used herein, is an artificial entity binding specifically to an affinity tag, typically a chemically synthesized modification or a recombinant protein or peptide attached to a molecule of interest. The ligand to an affinity tag depends on the type of affinity tag chosen and may be selected from the group consisting of His, immobilized nickel, glutathione, chitin, 18A, ACP, Aldehyd, Avi, BCCP, Calmodulin, Chitin binding protein, E-Tag, ELK16, FLAG, flash, poly glutamate, poly aspartate, GST, GFP, HA, Isope, maltose binding protein, myc, nus, NE, ProtA, ProtC, Tho1d4, S-Tag, SnoopTag, SpyTag, SofTag, Streptavidin, Strep-tag II, T7 Epitope Tag, TAP, TC, Thioredoxin, Ty, V5, VSV, biotin, Xpress Tag and a recombinant antibody binding to the ligand to an affinity tag.

According to the present invention, a mammalian antibody binding specifically to ZSCAN5 is provided, preferably in a liquid comprising one or more, more preferably all from the group comprising an artificial buffer, a preservative and an artificial anticoagulant and/or from the group comprising blood such as whole blood, plasma, serum and CSF. An artificial buffer is a buffer which is synthetic and/or may not occur in the body of the patient or at least at concentrations well below the concentration used. The buffer may be selected from the group consisting of Tris, phosphate, Tricine, acetate, MOPS, MES, carbonate, citrate and HEPES. In a preferred embodiment, the term "preservative" as used herein, refers to a substance inhibiting microbial growth and/or chemical degradation in a liquid solution and may preferably be selected from the group consisting of azide, lactic acid, nitrate, nitrite, antibiotics, a protease inhibitor and ethanol.

Various methods or uses according to the invention can be conducted with a sample from a subject or patient as described herein. These methods or uses can also be characterized as *"in vitro"* methods or *"in vitro"* uses.

In a preferred embodiment, the term "chemical solution reactive with a detectable label" refers to a compound in a liquid which, upon exposure to the detectable label, emits a detectable signal. The solution may comprise a chromogenic substrate of an enzymatically active label. For example, 3,3', 5,5' tetramethylbenzidine/H₂O₂ may be used if the label is a peroxidase. The solution may comprise a small inorganic or organic compound capable of reacting with a chemiluminescent label. In the case of an acridinium ester, a mixture of H₂O₂ and sodium hydroxide is frequently used as the chemical solution. Various other chemical solutions and detectable labels are known in the art (Weeks, I., Beheshti, I., McCapra, F., Campbell, A. K., Woodhead, J. S. (1983) Acridinium esters as high specific activity labels in immunoassay. Clin Chem 29: 1474-1479), Thermo Scientific Pierce Antibody Production and Purification Technical Handbook, Version 2, www.thermoscientific.com).

In a preferred embodiment, the methods, uses, mammalian antibodies, carriers and kits described herein are for diagnosing a disease and/or for aiding in the diagnosis of a disease. In a preferred embodiment, the term "diagnosis", as used herein, is to be understood in its broadest possible sense and may refer to any kind of procedure aiming to obtain information instrumental in the assessment whether a patient, known or an anonymous subject from a cohort, suffers or is likely or more likely than the average or a comparative subject, the latter preferably having similar symptoms, to suffer from a certain disease or disorder in the past, at the time of the diagnosis or in the future, to find out how the disease is progressing or is likely to progress in the future or to evaluate the responsiveness of a patient or patients in general with regard to a certain treatment, for example the administration of immunosuppressive drugs, or to find out whether a sample is from such a patient. Such information may be used for a clinical diagnosis but may also be obtained by an experimental and/or research laboratory for the purpose of general research, for example to determine the proportion of subjects suffering from the disease in a patient cohort or in a population. In other words, the term "diagnosis" comprises not only diagnosing, but also prognosticating and/or monitoring the course of a disease or disorder, including monitoring the response of one or more patients to the administration of a drug or candidate drug, for example to determine its efficacy. While the result may be assigned to a specific patient for clinical diagnostic applications and may be communicated to a medical doctor or institution treating said patient, this is not necessarily the case for other applications, for example in diagnostics for research purposes, where it may be sufficient to assign the results to a sample from an anonymized patient. Thus, in some embodiments, the person to be diagnosed, *i.e.,* the "subject" or "patient", is an anonymous blood donor whose blood may be donated or used to obtain therapeutically or diagnostically useful antibodies. The term "diagnosis" also refers to negative diagnosis, *i.e.,* the case where no mammalian antibodies specifically binding to ZSCAN5 are found in a sample from a patient. In these embodiments, the absence of mammalian antibodies specifically binding to ZSCAN5 indicates that the patient may suffer from a disease other than a disease associated with the presence of said antibodies, as described herein. In a preferred embodiment, a disease associated with the presence of an antibody binding specifically to ZSCAN5 is an autoimmune disease, such as a neurological autoimmune disease, as described herein. Thus, in one embodiment, "diagnosis" also includes the case where the absence of a mammalian antibody specifically binding to ZSCAN5 helps excluding certain diseases, such as the diseases associated with the presence of such antibody, as described herein, which may lead to the indirect diagnosis of another disease. In another preferred embodiment, the detection of a mammalian antibody binding specifically to ZSCAN5 is considered to imply a definitive diagnosis of a neurological autoimmune disease because of the presence of the antibody.

Thus, in a preferred embodiment, the term "diagnosis", "diagnosing" or "diagnostic" or similar terms encompasses diagnosis, prognosis, theragnosis and monitoring in an autoimmune disease, preferably a neurological autoimmune disease, such as those described herein, and/or a cancer, such as those described herein. As used herein, the term "theragnosis" refers to the identification, for example by diagnostic methods, of patients who might benefit from a particular therapy and, optionally, the subsequent treatment of said patients.

In a preferred embodiment, the methods and products according to the present invention may be used for interaction studies, including determining whether a drug candidate or other compound may interfere with the binding of a mammalian antibody binding specifically to ZSCAN5 or may affect any downstream process or the strength of its binding to its target. In a preferred embodiment, they may be used for monitoring the immune response, more preferably the emergence and/or titer of antibodies to ZSCAN5, following the administration of an immunogenic composition comprising at least one polypeptide comprising at least one epitope of ZSCAN5 or an immunogenic variant thereof, for example to a mammal, which may be a mammal other than a human such as a laboratory animal.

In a preferred embodiment, the methods and products may be used for providing reagents such as a mammalian antibody to ZSCAN5 which may serve as a positive control or a calibrator for a diagnostic or analytical test or for developing and/or validating a diagnostic or analytical test. In a preferred embodiment, the term "validating", as used herein, refers to a procedure for establishing an assay in a specific environment based on a previously known principle and confirming that it yields useful results. For example, while this application discloses the usefulness of a mammalian antibody to ZSCAN5 as a marker for a diagnosis or for aiding in the diagnosis, a clinical or research laboratory may need to confirm the diagnostic value of the results before routinely using the test for their patients or a new group of patients, for example a group of animals, which may not have been known previously to suffer from a disease or condition.

In another preferred embodiment, the methods and products according to the present invention may be used for determining the concentration of an antibody binding specifically to ZSCAN5. In a more preferred embodiment, said antibody is a mammalian antibody from a patient suffering from an autoimmune disease such as a neurological autoimmune disease. In another preferred embodiment, said antibody is a recombinant antibody which binds to ZSCAN5, but is recognized by a secondary antibody binding specifically to human IgG class antibodies, preferably IgG1, IgG2, IgG3 and IgG4 isotypes. In a more preferred embodiment, such a concentration needs to be determined for the purposes of research, for the preparation or for monitoring the quality of reagents, animal models or devices that may or may not be used for the diagnosis of a neurological autoimmune disease or for aiding in such diagnosis.

In many cases the mere detection of the mammalian antibody, in other words determining whether or not detectable levels of the antibody are present in the sample, is sufficient for the diagnosis. In a more preferred embodiment, this may involve determining whether the concentration is at least 10%, preferably 20%, 50%, 100%, 200%, 500%, 1,000%, 2,000%, 2,500%, 5,000%, 10,000%, 20,000%, 50,000%, 100,000%, 1,000,000% or 10,000,000% higher than the concentration of the antibody of interest found in the average healthy subject, preferably wherein the antibody is not detectable in the average healthy subject. If the antibody can be detected, this will be information instrumental for the clinician's diagnosis. It may indicate an increased likelihood that the patient suffers from a disease.

The person skilled in the art will appreciate that a clinician does usually not arrive at the conclusion whether or not the patient suffers or is likely to suffer from a disease, condition or disorders solely on the basis of a single diagnostic parameter, but needs to take into account other aspects, for example the presence of other antibodies, markers, blood parameters, clinical assessment of the patient's symptoms or the results of medical imaging or other non-invasive methods such as polysomnography, to arrive at a conclusive diagnosis. See Baenkler H. W. (2012), General aspects of autoimmune diagnostics, in Renz, H., Autoimmune diagnostics, 2012, de Gruyter, page 3. The value of a diagnostic agent or method may also reside the possibility to rule out one disease, thus allowing for the indirect diagnosis of another. In a preferred embodiment, the meaning of any symptoms or diseases referred to throughout this application is in line with the person skilled in the art's understanding as of the filing date or, preferably, earliest priority date of this application as evidenced by textbooks and scientific publications. In a preferred embodiment, the methods or uses or products are not used, taken alone, to arrive at a definite, final diagnosis.

In a preferred embodiment, any information or data demonstrating the presence or absence of the antibody may be communicated to the patient or a medical doctor treating the patient orally, preferably by telephone, in a written form, preferably fax or letter, or in an electronic form *via* fax or *via* the internet, for example as an email or text message.

The inventive teachings, such as the methods, uses, antibodies, carriers and kits described herein, may also be used in a method for preventing or treating a disease, preferably after a diagnosis according to the present invention, comprising the steps a) reducing the concentration of mammalian antibodies binding to ZSCAN5 in the subject's blood and/or b) administering one or more immunosuppressive pharmaceutical substances, preferably selected from the group consisting of rituximab, prednisone, methylprednisolone, cyclophosphamide, mycophenolatemofetil, intravenous immunoglobulin, tacrolimus, cyclosporine, methotrexate and azathioprine.

According to the present invention, the presence of an antibody may be determined in a qualitative or a quantitative manner. In a preferred embodiment, the term "detecting in a quantitative manner", as used herein, means that not only the presence of an antibody is detected, but that a result is obtained that includes information regarding the absolute or relative amount of the antibody in the sample. In a more preferred embodiment, a value representing an absolute concentration is obtained. In another more preferred embodiment, a value representing a relative concentration or change of concentration is obtained. In another preferred embodiment, also referred to as "semi-quantitative" approach, the concentration of the antibody is placed in one of several groups, most preferably a concentration window meaning that it is virtually absent, a concentration window meaning that a borderline result is obtained and a concentration window meaning that the antibody is present. A further distinction into categories such as "weak positive" or "strong positive" signal is possible.

The term "isolated" as used herein means that the molecule referred to as being isolated is free or essentially free of at least one component as it is found in nature. For example, the molecule, such as a mammalian antibody, may be free or essentially free from some or all components as it is found in its natural environment. Such components may comprise, for example in the case of a mammalian antibody, erythrocytes, leucocytes, thrombocytes, plasma, proteins, nucleic acids, salts, lipids, and nutrients.

The method according to the present invention is preferably an *in vitro* method.

According to the present invention, a polypeptide, such as the at least one polypeptide comprising at least one epitope of ZSCAN5 or a variant thereof, may be a recombinant polypeptide. In a preferred embodiment, the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In other preferred embodiments, a polypeptide provided or used according to the present invention such as a polypeptide comprising at least one epitope of ZSCAN5 or a variant thereof or an antibody is an isolated polypeptide, wherein the term "isolated" may mean that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier is pure.

Patients having mammalian antibodies to ZSCAN5 may suffer from a variety of cancers comprising mamma carcinoma, lung carcinoma (NSCLC), cerebral metastases and melanoma. In a preferred embodiment, the term "cancer", as used herein, refers to a group of diseases involving abnormal cell growth with the potential to invade or spread to other parts of the body.

In a preferred embodiment, the term "mamma carcinoma" or "breast cancer", as interchangeably used herein, is defined as cancer which originates in the breast, whereas the cells have malignant form and structure, the ability for uncontrolled growth and the potential or ability to invade or metastasize. In a preferred embodiment, the breast cancer may spread to other organs, such as lymph nodes. In further preferred embodiments, the breast cancer is invasive and may be metastatic.

In a preferred embodiment, the term "non-small cell lung carcinoma (NSCLC)", as used herein, includes epidermoid carcinoma cells, adenocarcinoma cells, and large cell undifferentiated carcinoma cells. The most common symptoms associated with NSCLC are coughing (including coughing up blood), weight loss, shortness of breath, and chest pains.

In a preferred embodiment, the term "cerebral metastases", as used herein, refers to tumors, which originate from outside the Central Nervous System (CNS, such as brain and spinal cord) and spread to it via the blood stream or directly invade from neighboring tissues. Metastatic tumors are the commonest tumors that affect the brain and spinal cord.

In a preferred embodiment, the term "melanoma", as used herein, refers to a malignant tumor of melanocytes. Melanocytes are cells that produce the dark pigment, melanin, which is responsible for the color of skin. They predominantly occur in skin but are also found in other parts of the body, including the bowel and the eye. Melanoma can occur in any part of the body that contains melanocytes.

Additional background and definitions related to cancers and their diagnoses or differential diagnoses including the detection of antibodies may be taken from neurology textbooks available at the earliest priority date or filing date of this publication such as Kaye, Textbook of Medical Oncology, 3rd edition, Taylor & Francis; Shoenfeld, Meroni and Gershwin, Autoantibodies 3rd edition, Elsevier, in particular Part 11 including Chapter 76; and Darnell and Posner, Paraneoplastic Syndromes, Oxford University Press, 2011.

Patients having antibodies to ZSCAN5 or a variant thereof may suffer from a variety of neurological autoimmune diseases comprising PNS, dementia, cognitive decline, psychotic symptoms, acoustic hallucinations, neuropsychiatric symptoms, seizures, epilepsy, encephalitis, such as limbic encephalitis or autoimmune encephalitis, neuromyelitis optica spectrum disorder (NMOSD), neuromyotonia, neuropathy, Multiple Sclerosis, peripheral polyneuropathy, Guillain-Barré syndrome (GBS) symptoms, tetraparesis, cerebellar/brainstem syndrome, hearing loss, symmetric maculopathy, cerebellopontine lesion extending into spinal cord, personality changes, cognitive impairment and affect disorder, ataxia, such as gait and stand ataxia, multiple cerebral ischemia, and cancers such as carcinoma, cerebral metastases, leukemia and melanoma, preferably PNS, dementia, cognitive decline, encephalitis, such as limbic encephalitis or autoimmune encephalitis, neuromyelitis optica spectrum disorder (NMOSD), neuromyotonia, neuropathy, Multiple Sclerosis, ataxia, such as gait and stand ataxia, epilepsy and neuropsychiatric symptoms.

In a preferred embodiment, the term "ataxia", as used herein, refers to lack of voluntary coordination of muscle movements that can include gait abnormality, speech changes, and abnormalities in eye movements. Ataxia is a clinical manifestation indicating dysfunction of the parts of the nervous system that coordinate movement, such as the cerebellum.

In a preferred embodiment, the term "polyneuropathy", as used herein, refers to a disease affecting peripheral nerves (peripheral neuropathy) in roughly the same areas on both sides of the body, featuring weakness, numbness, and burning pain.

In a preferred embodiment, the term "encephalitis", as used herein, refers to an inflammation of the brain, with symptoms including reduced or alternation in consciousness, personality changes, psychotic delusions, rigidity, headache, fever, confusion, a stiff neck, and vomiting. Complications may include seizures, hallucinations, trouble speaking, memory problems, and problems with hearing. The disease may be the result of an infection or may be an autoimmune disease, hence the detection of an antibody according to the invention may be used to distinguish these two types of encephalitis.

In a preferred embodiment, the term "neuropsychiatric symptoms", as used herein, refers broadly to non-cognitive disturbances, including depression, psychosis, agitation, aggression, apathy, sleep disturbances, and disinhibition. Neuropsychiatric symptoms are known to accompany decline of cognitive ability such as dementia and appear in almost all cases of dementia.

In a preferred embodiment, the term "dementia", as used herein, refers broadly to any disorder, disease, or syndrome characterized by an abnormal high and progressive loss of functional capacity of the brain. Hallmarks of dementia include impairment of several core mental functions, including memory, communication and language, ability to focus and pay attention, reasoning and judgment, and visual perception. People with dementia may have problems with short-term memory, keeping track of a purse or wallet, paying bills, planning and preparing meals, remembering appointments or traveling out of the neighborhood. Dementia may be progressive, meaning symptoms start out slowly and gradually get worse. Dementia may be determined using standard clinical procedures, with the degree of dementia being defined by the score in the Mini Mental State Examine (MMSE), as detailed in Folstein M.F., Folstein S.E. and McHugh P.R., J Psychiatry Res., 12:189-198 (1975).

In a preferred embodiment, the term "ischemia", as used herein, refers to a condition resulting from a decrease or lack of blood flow and oxygen to a part of the body such as the brain, heart, or other tissue. Ischemic injury refers generally to the damage to a tissue that is distal or otherwise effected by the loss of blood flow and oxygen. Ischemic injury is often a result of the lack of oxygen and fluids, but also includes inflammatory cascades. For example, ischemia and ischemic injury can occur as a result of cardiac, pulmonary or brain injury, organ transplantation or surgical procedure, or a disease or disorder (e.g., Sickle Cell Anemia or Sickle Cell Disease). In more preferred embodiments, the ischemia is multiple cerebral ischemia.

In a preferred embodiment, the term "Paraneoplastic Neurological Syndrome" (PNS), as used herein, refers to neurological syndromes associated with the presence of a cancer associated with tumors which presents an antigen which is normally exclusive to the nervous system. As a result, an antibody binding specifically to the neurological antigen is produced which may then damage the nervous system. Manifestations of PNS include, but are not limited to encephalitis, typically associated with seizures, psychiatric manifestations such as hallucinations, anxiety and depression, cerebellar symptoms, such as ataxia, nystagmus and dysarthria, opsoclonus-myoclonus and sensory neuronopathy; and Lambert-Eaton myasthenic syndrome. It should be mentioned that PNS-associated tumors are often small, grow slowly and may not yet be detectable when neurological syndromes surface. PNS may be associated with one or more antibodies, which may bind specifically to an autoantigen from the group comprising NMDAR, Lgl1, AMPA1, AMPA2, CASPR2, GABA B, GABA A, DPPX, IGLON5, Hu, Yo, CRMP5, Ri, Ma2, Amphiphysin, Recoverin, RGS8, DAGLA, NSF, STX1B, DNM1 and VAMP2, Hu, Ri, Ma, Anna-3, Zic-4, SOX1, Yo, PCA2, Tr and glutamic acid decarboxylase.

Additional background and definitions related to neurological syndromes and symptoms and their diagnoses or differential diagnoses including the detection of antibodies may be taken from neurology textbooks available at the earliest priority date or filing date of this publication such as Simon, Greenberg, Aminoff, Clinical Neurology, 7th edition, 2009, McGraw; Shoenfeld, Meroni and Gershwin, Autoantibodies 3rd edition, Elsevier, in particular Part 11 including Chapter 76; and Darnell and Posner, Paraneoplastic Syndromes, Oxford University Press, 2011.

The methods described herein for determining the presence of a mammalian antibody binding to ZSCAN5 in a sample can be combined with methods for determining the presence of other antibodies, the presence of which is associated with the occurrence of the diseases described herein or of similar diseases. For example, at least one polypeptide comprising at least one epitope of ZSCAN5, or a variant thereof, as described herein, can be used in conjunction with one or more polypeptides or variants thereof binding to other antibodies, the presence of which is associated with the occurrence of the diseases described herein or of similar diseases. For example, a device comprising at least one polypeptide comprising at least one epitope of ZSCAN5, or a variant thereof, such as the device of the present invention, may comprise further polypeptides or variants thereof binding to other antibodies, the presence of which is associated with the occurrence of the diseases described herein or of similar diseases. Such device may be used to analyze a sample from a subject such as a patient for the presence of any antibody, the presence of which is associated with the occurrence of the diseases described herein or of similar diseases. In the case that a method using such a device or using several devices, wherein each device comprises at least one polypeptide comprising at least one epitope of ZSCAN5 or a variant thereof, or a polypeptide binding to another antibody, which does not bind to ZSCAN5, the presence of which is associated with the occurrence of the diseases described herein or of similar diseases, determining the presence of any antibody binding to any of the assayed polypeptides or variants thereof may be sufficient for a skilled person to conclude on the presence of a certain disease or to limit the suspected disease to certain diseases or to aid in the diagnosis of a disease, as described herein, or to gain information about the risk of a subject to suffer in the present, past or future from or to develop a disease as described herein. Thus, under certain circumstances, it may be sufficient to determine the presence of either an antibody binding to ZSCAN5 or of any other antibody, which does not bind to ZSCAN5, but the presence of which is associated with the occurrence of the diseases described herein or of similar diseases, without getting information or reaching a conclusion which specific antibody is present in a given sample, as long as the presence of said antibodies is associated with the occurrence of the same or similar disease(s). Consequently, determining the presence of a mammalian antibody binding to ZSCAN5 may comprise determining the presence of any antibody, the presence of which is associated with the occurrence of the diseases described herein as being associated with the presence of a mammalian antibody to ZSCAN5 or of similar diseases.

A person skilled in the art will understand that under certain circumstances, it may be instrumental to first analyze the clinical symptoms of a patient having or suspected of having a disease which is or is suspected of being associated with the presence of an antibody binding to ZSCAN5. Depending on the symptoms, grade of impairment and other factors known in the art, a clinician or another medical or scientific personal may then determine whether the antibody titer of the subject to be analyzed is increased compared to a healthy subject or compared to the titer of the subject to be analyzed prior to the onset of symptoms or compared to the average. Subsequently or independently, one or more methods as described herein such as for determining the presence of a mammalian antibody binding to ZSCAN5 may be conducted to determine the presence of such antibody and/or to determine the presence of any antibody, the presence of which is associated with a disease as described herein. Thus, determining the presence of a mammalian antibody binding to ZSCAN5 may be used as a preinvestigation or as one investigation amongst others allowing a clinician to reach a conclusion whether or not a subject suffers or is likely to suffer in the present, past or future from a disease. Determining the presence of a mammalian antibody binding to ZSCAN5 may also be used to distinguish between drug or alcohol use or abuse or the presence of an infectious disease, or the presence of a psychiatric condition, which is not autoimmune in nature, and the presence of an autoimmune disease, as described herein.

In certain embodiments, it may be instrumental to determine the Ig class of an antibody binding to ZSCAN5. Thus, in one embodiment, determining the presence of a mammalian antibody binding to ZSCAN5 comprises determining the Ig class of said antibody. In other embodiments, determining the presence of a mammalian antibody binding to ZSCAN5 does not comprise determining the Ig class of said antibody. Likewise, determining the presence of a mammalian antibody binding to ZSCAN5 of a certain Ig class may comprise determining the presence of antibodies binding to ZSCAN5 of other Ig classes. Determining the presence of a mammalian antibody binding to ZSCAN5 of a certain Ig class may also comprise determining the absence of said antibody, e.g. by obtaining a negative result which may indicate that no antibody binding to ZSCAN5 is present in an assayed sample.

As part of a diagnosis relating to the neurological syndromes associated with antibodies specifically binding to ZSCAN5, the clinician will initially consider a range of tests and risk factors which may point them either to autoimmune disease or infectious disease for many of the conditions associated with the presence of a mammalian antibody binding specifically to ZSCAN5 (Lancaster, J Clin Neurol. 2016 Jan;12(1) https://doi.org/10.3988/jcn.2016.12.1.1, Lee and Lee, The Laboratory Diagnosis of Autoimmune Encephalitis, Journal of Epilepsy Research 6 (2), 45), preferably encephalitis, more preferably limbic encephalitis or autoimmune encephalitis, NMOSD, PNS, dementia, Multiple Sclerosis. Detection of a mammalian antibody binding specifically to ZSCAN5 will then confirm an autoimmune background, while the absence of a mammalian antibody to ZSCAN5 will tempt the clinician to consider autoimmune diseases associated with other antibodies. However, typically the presence or absence of a range of antibodies will then be detected, and a negative result will be a pointer to infectious diseases. In one embodiment, a negative result may also be interpreted as a pointer to alcohol or drug abuse or the presence of a psychiatric condition, which is not autoimmune in nature.

According to the present invention, a kit is provided, comprising the cell or the carrier and further comprising one or more, preferably all reagents from the group consisting of a secondary antibody, preferably labeled with a detectable label, a washing solution, a positive control, a negative control, a detergent, a cover glass, a mounting medium and a physiological salt solution, preferably PBS, or salt required to prepare it. In a preferred embodiment, the positive control is a diluted sample, preferably whole blood, more preferably serum or CSF, from a patient suffering from a neurological autoimmune disease or a monoclonal or polyclonal antibody binding specifically to ZSCAN5. The negative control may be a diluted sample from a healthy subject, for example a blood donor. The kit may comprise instructions how to carry out the assay. Preferably, the secondary antibody is a secondary antibody binding specifically to IgG class antibodies, preferably human IgG class antibodies, more preferably to the constant region of a human IgG class antibody. In other preferred embodiments, said secondary antibody may be labeled (as described above).

In a preferred embodiment, the present invention provides a use of the cell, the polypeptide, the carrier or the mammalian antibody for the manufacture of a kit or a composition, optionally for the diagnosis or aiding in the diagnosis of a disease.

In a preferred embodiment, any method or use according to the present invention may be intended for a non-diagnostic use, i.e. determining the presence of a mammalian antibody binding specifically to ZSCAN5, for a use other than diagnosing a patient. For example, the method or use may be for testing *in vitro* the efficiency of a medical device designed to remove a mammalian antibody from a patient's blood, wherein the testing is performed on a liquid other than patient's blood. After the use of the medical device with a patient, its capacity to remove mammalian antibody may be checked by running a solution comprising antibody binding specifically to ZSCAN5 through the device, followed by use of the method according to the present invention to confirm that less or no antibody is in the solution that has been passed through the device, i.e. showing that the device has still the capacity to remove antibody from the solution.

According to the present invention, the method or use may be used for testing the efficacy of a drug candidate which may be used to treat patients suffering from or likely to suffer from a neurological autoimmune disease. Such a drug candidate may be any molecule capable of interfering with the interaction between ZSCAN5 or variant thereof and the corresponding mammalian antibody.

In another preferred embodiment, the method may be for confirming the reliability of a diagnostic assay, e.g., for the diagnosis of a disease associated with the presence of a mammalian antibody binding to ZSCAN5 or a variant thereof, and may involve detecting an antibody binding specifically to ZSCAN5 in a solution, which is not a sample from a patient who requires a diagnosis but is known to comprise an antibody binding specifically to ZSCAN5, preferably at a known concentration. For example, it may be a recombinant antibody or a sample diluted in a dilution buffer such as PBS from an anonymous patient whose identity cannot be traced back. Alternatively, the solution may be a negative control not comprising the antibody binding specifically to check the background. Such method may be run in parallel with, after or before a diagnostic method. In a preferred embodiment, any method or use according to the present invention may be intended for generating an autoantibody profile, preferably for detecting a disease in a mammal, preferably a human.

In a preferred embodiment, any method or use according to the present invention may be for identifying a subject at risk of suffering from or developing a disease, such as an autoimmune disease described herein, and/or cancer, such as a cancer described herein.

In a preferred embodiment, the method may be for detecting a mammalian antibody binding specifically to ZSCAN5 in a solution which is not a sample from a mammal to be diagnosed or for the purpose of providing a diagnosis. In a preferred embodiment, the problem underlying the present invention is solved by a method comprising the step contacting a device comprising a solid phase on which at least one polypeptide comprising at least one epitope of ZSCAN5 or a variant thereof is immobilized with a buffered solution comprising a mammalian antibody binding specifically to ZSCAN5, which solution is preferably not a sample from a patient in need of a diagnosis, wherein preferably
a) the concentration of the antibody in the solution is known, and/or
b) the antibody is a recombinant antibody and/or
c) the medical or diagnostic device is contacted with two or more solutions comprising the antibody, wherein the two or more solutions have a different concentration of the antibody, and/or
d) the antibody is recognized by secondary antibodies binding specifically to IgG antibodies,
followed by detection of a signal which indicates whether or not the antibody has bound to the polypeptide, optionally a signal relating to the concentration of the antibody in the solution or solutions.

In a preferred embodiment, the present invention provides an apparatus for analyzing a sample from a patient to detect a mammalian antibody against ZSCAN5, indicating an increased likelihood of a neurological autoimmune disease or of developing it, comprising:
a. a carrier, which contains a means for capturing the antibody from the sample when the sample is contacted with the carrier, preferably wherein the means is the cell or at least one polypeptide and the carrier is the carrier according to the present invention,
b. a detectable means capable of binding to the antibody captured by the carrier when the detectable means is contacted with the carrier, wherein the detectable means is preferably a labeled secondary antibody capable of binding to the antibody captured on the carrier,
c. optionally a means for removing any sample from the carrier and the detectable means, preferably by washing;
d. a detecting device for detecting the presence of the detectable means and converting the results into an electrical signal, for example a fluorescence reader or a fluorescence microscope connected with a software capable of recognizing a pattern characteristic of a stained cell overexpressing a polypeptide comprising at least one epitope of ZSCAN5 or a variant thereof in an image of the cell taken by the fluorescence reader or camera, and
optionally a means for receiving the electronical signal from the detecting device and determining if the level of the signal is indicative of an increased likelihood of having or developing a disease, by comparing with the patterns characteristic of wild type or non-stained cells, preferably by a mock-transfected cell or cells not positively stained by an antibody binding specifically to ZSCAN5 on the same carrier, or an input reference value obtained with samples from healthy subjects or by comparing the level of signal obtained with one sample with the level of signal obtained with a second sample obtained at a later time point, preferably at least one month later.

According to the present invention, a device for removing a mammalian antibody binding specifically to ZSCAN5 from blood, preferably serum of a patient suffering from a neurological autoimmune disease, wherein the device comprises a carrier with a solid phase on which at least one polypeptide comprising at least one epitope of ZSCAN5 or a variant thereof is immobilized is provided as is an ex *vivo* method for removing a mammalian antibody binding specifically to ZSCAN5 from a patient. A device on which at least one polypeptide comprising at least one epitope of ZSCAN5 or a variant thereof or a secondary antibody or protein capturing all IgG class antibodies, among them IgG class antibodies to ZSCAN5, is immobilized may be used. Suitable methods are described in Eisei Noiri and Noria Hanafusa, The Concise Manual of Apharesis Therapy, Springer Tokyo, 2014. Hamilton, P., Kanigicherla, D., Hanumapura, P., Walz, L., Kramer, D., Fischer, M., Brenchley, P., and Mitra, S. (2018) J. Clin. Aph. 33(3), 283-290. Another method is disclosed in EP3477300.

The term "at least one" comprises "exactly one" and "more than one". For example, a polypeptide comprising at least one epitope may refer to a polypeptide comprising exactly one epitope but it may also refer to a polypeptide comprising more than one epitope, such as 2, 3, 4, 5, 6, 7, 8, 9, 10, 11, 12, 13, 14, 15, 20, 25, 30, 35, or even more epitopes. In some embodiments, the term "one or more" comprises "at least one". In some embodiments, "one or more" and "at least one" are interchangeably.

In some embodiments, the term "preferably" comprises "optionally". In some embodiments, "preferably" and "optionally" are interchangeably.

The present invention is further illustrated by the following non-limiting examples from which further features, embodiments, aspects and advantages of the present invention may be taken.

### Figures

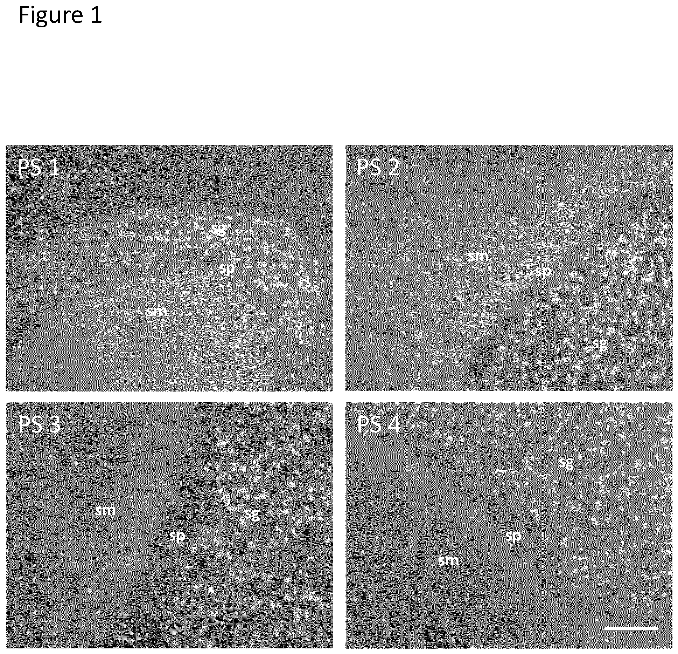

**Figure 1****. Immunofluorescence staining of neuronal tissues.**
   Cryosections of rat hippocampus and cerebellum as well as primate cerebellum were incubated with patient serum 1, 2, 3, and 4 (1:32 dilution, green) in the first step, and with Alexa488-labelled goat anti-human IgG in the second step. A spotty staining of the granular layer and granular staining of the molecular layer of primate cerebellum was observed but no reaction with rat neuronal tissues was observed. sm: stratum moleculare, sg: stratum granulosum, sp: stratum purkinjense, scale bar 100 µm.
**Figure 2****. Epitope characterization.**
   PhIP-Seq analysis revealed that the index samples bound peptides in ZSCAN5B, ZSCAN5C (patient sera 1-4), and ZSCAN5A (only patient sera 1 and 3), indicating the epitope must be located between AS 272 - 360 for ZSCAN5B and AS 271 - 360 for ZSCAN5C and ZSCAN5A.
**Figure 3****. Neutralization of antibody reaction on neuronal tissues.**
   Patient serum 2 (1:32, green) was pre-incubated with extracts (1:2) of HEK293 cells transfected with empty control vector or with plasmids harboring ZSCAN5B cDNA in the first step, and with Alexa488-labelled goat anti-human IgG in the second step. The extract containing ZSCAN5B greatly abolished the immune reaction of the serum on monkey cerebellar sections. Nuclei were counterstained by incubation with TO-PRO-3 iodide (blue). Scale bar: 100 µm.

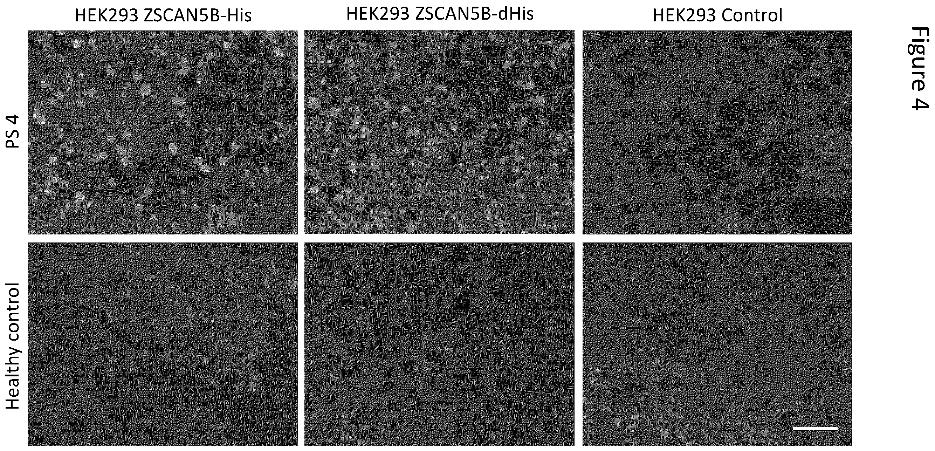

**Figure 4****. Verification of ZSCAN5 as the novel autoantigen by indirect immunofluorescence with the recombinant antigen.**
   Indirect immunofluorescence assay using acetone-fixed ZSCAN5B-His, ZSCAN5B dHis or empty vector transfected HEK293 cells incubated with patient serum 4 (1:100 dilution) or a healthy control (1:10 dilution) and with anti-human IgG-FITC in the second step. Scale bar 100 µm.

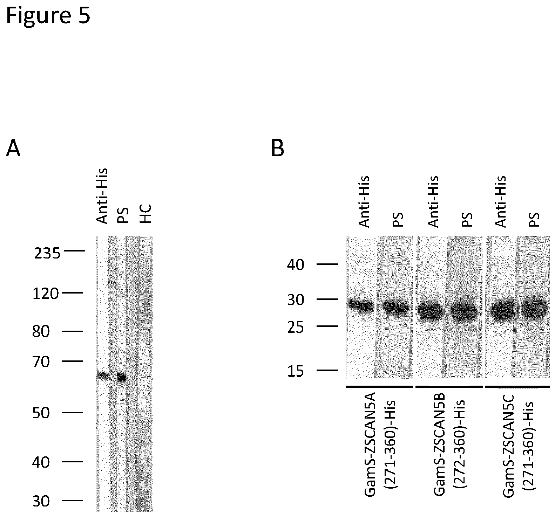

**Figure 5****. Verification of ZSCAN5 as the novel autoantigen by Western Blot with the recombinant antigen.**
   A) Immunoblot with recombinantly expressed ZSCAN5B-His purified from E. coli, and incubated with anti-His antibody, sera of 13 patients (PS, one representative blot is depicted) or 15 healthy control sera (HC, one representative blot is depicted) (1 :200). B) Immunoblot with epitope-containing fragments of ZSCAN5 expressed in a cell-free protein expression system incubated with sera of four patients (PS, one representative blot is depicted per ZSCAN paralog).

### Sequences

The present invention comprises a range of novel nucleic acid and amino acid sequences, more specifically
**SEQ ID NO:1 (fragment of ZSCAN5)**
   ESDPIQALRKLTELCHLWLRPDLHTKEQILDMLVMEQFMISMPQELQVLV
**SEQ ID NO:2 (fragment of ZSCAN5)**
   NGVQSCKDLEDLLRNNRRPKKWS
**SEQ ID NO:3 (fragment of ZSCAN5)**
   ASVENVDADTPSACVVEREA
**SEQ ID NO:4 (fragment of ZSCAN5)**
   SISQEEPQGEATPVGNRESPGQA
**SEQ ID NO:5 (ZSCAN5A)** 
MAANCTSSWSLGESCNRPGLELPRSMASSETQLGNHDVDPEISHVNFRMFSCPKESDPIQ
ALRKLTELCHLWLRPDLHTKEQILDMLVMEQFMISMPQELQVLVMMNGVQSCKDLEDLLRN
NRRPKKWSVVTFHGKEYIVQDSDIEMAEAPSSVRDDLKDVSSQRASSVNQMRPGEGQAHR
ELQILPRVPALSRRQGEDFLLHKSIDVTGDPKSLRPKQTLEKDLKENREENPGLTSPEPQLP
KSPTDLVRAKEGKDPPKIASVENVDADTPSACVVEREASTHSGNRGDALNLSSPKRSKPDA
SSISQEEPQGEATPVGNRESPGQAGMNSIHSPGPASPVSHPDGQEAKALPPFACDVCEKR
FTCNSKLVIHKRSHTGERLFQCNLCGKRFMQLISLQFHQRTHTGERPYTCDVCQKQFTQKS
YLKCHKRSHTGEKPFECKDCKKVFTYRGSLKEHQRIHSGEKPYKCSKCPRAFSRLKLLRRH
QKTHPEATSQ
**SEQ ID NO:6 (ZSACN5B)** 
MAANWTLSWGQGGPCNSPGSDTPRSVASPETQLGNHDRNPETWHMNFRMFSCPEESDP
IQALRKLTELCHLWLRPDLHTKEQILDMLVMEQFMISMPQELQVLVKVNGVQSCKDLEDLLR
NNRRPKKWSIVNLLGKEYLMLNSDVEMAEAPASVRDDPRDVSSQWASSVNQMHPGTGQA
RREQQILPRVAALSRRQGEDFLLHKSIDVTGDPNSPRPKQTLEKDLKENREENPGLSSPEP
QLPKSPNLVRAKEGKEPQKRASVENVDADTPSACVVEREALTHSGNRGDALNLSSPKRSK
PDASSISQEEPQGEATPVGNRESPGQAEINPVHSPGPAGPVSHPDGQEAKALPPFACDVC
NKSFKYFSQLSIHRRSHTGDRPFQCDLCRKRFLQPSDLRVHQRVHTGERPYMCDVCQKRF
AHESTLQGHKRIHTGERPFKCKYCSKVFSHKGNLNVHQRTHSGEKPYKCPTCQKAFRQLG
TFKRHLKTHRETTSQ
**SEQ ID NO:7 (ZSCAN5C)** 
MAANCTSSWSLGESCNSPGSEPPQSMPSPATQLGNHDSDPETCHVNFRMFSCPKESDPI
QALRKLTELCHLWLRPDLHTKEQILDMLVMEQFMISMPQELQVLVMMNGVQSCKDLEDLLR
NNRRPKKWSVVSFLGKEYLMQESDVEMAEAPASVRDDPRHVSSQRTSSVNQMCPEEGQA
 
SQELQTLPRVPALFRRQEEDFLLPETTVMKGDPKALRPKPTLEKDLEEDREENPGLTSPEP
QLPNSPTGVVGAKEGKEPQKRASVENVDADTPSACVVEREASTHSGSRGDALNLRGLKRS
KPDATSISQEEPQGEATPVGNRESPGQAEINPVHSPGPAGPVSHPSGQEVKELLPFACEVC
GKRFKYRGKLAVHTRSHTGERLFQCNLCGKRFMQRIGLQFHQRTHTGERPYTCDICQKQF
TQKSYLKCHKRSHTGEKPFECKDCKKVFTYKANLKEHQRIHSGEKPHKCSKCPRAFGRPAT
LRRHQKTHREATSQ
**SEQ ID NO:8 (ZSCAN5 epitope)**
X1=T or absent
X2=P or absent
X12=S or L
X17=S or N
X25=S or R
X26=S or G
X27=P or L
X35=S or T
X59=G or E
X60=M or I
X62=S or P
X63=I or V
X70=S or G
X76=D or S
X80=A or V
X82=A or E
X84=P or L
X89=D or E
X91=C or absent 
XXSACVVEREAXTHSGXRGDALNLXXXKRSKPDAXSISQEEPQGEATPVGNRESPGQAXX
NXXHSPGPAXPVSHPXGQEXKXLXPFACXVX
SEQ ID NO:9 (ZSCAN5 fragment)
   MAANCTSSWSLGESCN
**SEQ ID NO:10 (ZSCAN5 fragment)** 
HVNFRMFSCPKESDPIQALRKLTELCHLWLRPDLHTKEQILDMLVMEQFMISMPQELQVLV
MMNGVQSCKDLEDLLRNNRRPKKWSVV
**SEQ ID NO:11 (ZSCAN5 fragment)**
   REENPGLTSPEPQLP
**SEQ ID NO:12 (ZSCAN5 fragment)**
   ASVENVDADTPSACVVEREASTHSG
**SEQ ID NO:13 (ZSCAN5 fragment)**
   RSHTGERLFQCNLCGKRFMQ
**SEQ ID NO:14 (ZSCAN5 fragment)**
   LQFHQRTHTGERPYTCD
**SEQ ID NO:15 (ZSCAN5 fragment)**
   CQKQFTQKSYLKCHKRSHTGEKPFECKDCKKVFTY
**SEQ ID NO:16 (ZSCAN5 fragment)**
   LKEHQRIHSGEKP
**SEQ ID NO:17 (ZSCAN5 fragment)**
   KCSKCPRAF
**SEQ ID NO:18 (ZSCAN5 fragment)**
   SDVEMAEAPASVRDDPR
**SEQ ID NO:19 (ZSCAN5 fragment)**
   AKEGKEPQKRASVENVDADTPSACVVEREA
**SEQ ID NO:20 (ZSCAN5 fragment)**
   SISQEEPQGEATPVGNRESPGQAEINPVHSPGPAGPVSHP
**SEQ ID NO:21 (ZSCAN5 fragment)**
   ALSRRQGEDFLLHKSIDVTGDP
**SEQ ID NO:22 (ZSCAN5 fragment)**
   RPKQTLEKDLKENREENPGL
**SEQ ID NO:23 (ZSCAN5 fragment)**
   THSGNRGDALNLSSPKRSKPDASSISQEEPQGEATPVGNRESPGQA
**SEQ ID NO:24 (ZSCAN5 fragment)**
   PVSHPDGQEAKALPPFACDVC
**SEQ ID NO:25 (ZSCAN5B, gene synthesis fragment 1, coding aa1-242)** 
ATACGTCTCACATGGCTGCAAATTGGACACTCTCATGGGGTCAGGGAGGACCCTGCAA
CAGCCCTGGGTCAGACACTCCACGGTCTGTGGCGTCCCCAGAAACTCAACTTGGAAAT
CACGACAGGAACCCTGAGACTTGGCACATGAATTTCAGGATGTTCAGCTGCCCAGAGG
AGTCGGACCCCATCCAGGCTCTGAGGAAACTCACTGAGCTGTGCCATCTGTGGCTGAG
GCCCGACCTCCACACCAAAGAGCAGATCCTGGACATGCTGGTGATGGAGCAGTTCATG
ATCTCCATGCCCCAGGAGCTCCAGGTCTTAGTCAAGGTGAACGGTGTGCAGAGCTGCA
AAGACCTGGAGGACCTGCTACGAAATAACAGAAGACCCAAGAAATGGTCTATAGTCAAC
TTGCTCGGCAAAGAATATCTTATGCTGAACTCAGATGTCGAGATGGCTGAAGCCCCCGC
CAGTGTCAGAGATGATCCGAGAGATGTGTCCAGCCAGTGGGCCTCCTCTGTGAACCAG
ATGCATCCGGGGACAGGCCAGGCCCGCCGAGAGCAGCAGATCCTGCCCAGGGTCGCT
GCACTGTCCAGGAGGCAGGGAGAGGACTTTCTGCTACACAAGAGTATTGACGTAACAG
GTGACCCAAACTCTCCGAGACCCAAGCAGACCTTGGAGAAGGATCTGAAGGAAAACAG
GGAAGAGAACCCAGGACTGTCATCCCCAGAGCCTCAGCTTAGAGACGTAT
**SEQ ID NO:26 (ZSCAN5B, gene synthesis fragment 2, coding aa243-495)** 
TCACGTCTCAGCTTCCAAAGAGTCCCAATCTGGTGAGAGCAAAGGAGGGGAAGGAACC
CCAGAAAAGAGCCTCTGTGGAAAATGTGGATGCTGACACACCTTCTGCCTGCGTTGTGG
AGAGAGAAGCTTTGACTCACAGCGGGAACAGAGGAGATGCTCTGAATCTGAGCAGTCC
CAAAAGAAGCAAACCAGATGCCTCCTCCATTTCCCAAGAAGAGCCTCAAGGAGAAGCCA
CACCTGTGGGCAACAGAGAATCCCCAGGACAAGCTGAGATCAATCCAGTTCATTCCCCA
GGCCCTGCGGGCCCGGTCAGTCACCCAGATGGCCAAGAAGCCAAGGCCCTGCCGCCC
TTTGCATGTGACGTGTGCAATAAATCATTTAAGTATTTTTCCCAGCTAAGCATCCACAGG
AGGTCACACACAGGAGACAGACCCTTTCAATGTGATCTCTGTCGGAAGCGCTTCTTGCA
GCCCTCAGACCTCCGAGTTCACCAGCGAGTCCACACTGGCGAGAGGCCCTACATGTGT
 
GACGTCTGCCAAAAGCGGTTCGCCCACGAGTCCACCTTACAGGGCCACAAGAGGATCC
ACACCGGGGAGAGGCCGTTTAAATGTAAATACTGCAGCAAAGTTTTCAGCCACAAGGG
GAACCTGAACGTTCACCAGCGCACCCACTCCGGAGAGAAGCCCTACAAATGTCCCACC
TGTCAAAAGGCCTTCCGTCAGCTGGGGACATTCAAGCGTCACCTGAAAACACACCGGG
AAACCACCTCCCAGTGATAATCGACGAGACGCAT
**SEQ ID NO:27 (sense ZSCAN5B-F3)**
   ATACGTCTCACATGGCTGCAAATTGGACACTC
**SEQ ID NO:28 (asense ZSCAN5B-F3)**
   ATACGTCTCATCGAGCTGGGAGGTGGTTTCCCGGTGTGTTTTC
**SEQ ID NO:29 (ZSCAN5B -PCR-fragment-3 without Stop-Codon)** 
TCACGTCTCAGCTTCCAAAGAGTCCCAATCTGGTGAGAGCAAAGGAGGGGAAGGAACC
CCAGAAAAGAGCCTCTGTGGAAAATGTGGATGCTGACACACCTTCTGCCTGCGTTGTGG
AGAGAGAAGCTTTGACTCACAGCGGGAACAGAGGAGATGCTCTGAATCTGAGCAGTCC
CAAAAGAAGCAAACCAGATGCCTCCTCCATTTCCCAAGAAGAGCCTCAAGGAGAAGCCA
CACCTGTGGGCAACAGAGAATCCCCAGGACAAGCTGAGATCAATCCAGTTCATTCCCCA
GGCCCTGCGGGCCCGGTCAGTCACCCAGATGGCCAAGAAGCCAAGGCCCTGCCGCCC
TTTGCATGTGACGTGTGCAATAAATCATTTAAGTATTTTTCCCAGCTAAGCATCCACAGG
AGGTCACACACAGGAGACAGACCCTTTCAATGTGATCTCTGTCGGAAGCGCTTCTTGCA
GCCCTCAGACCTCCGAGTTCACCAGCGAGTCCACACTGGCGAGAGGCCCTACATGTGT
GACGTCTGCCAAAAGCGGTTCGCCCACGAGTCCACCTTACAGGGCCACAAGAGGATCC
ACACCGGGGAGAGGCCGTTTAAATGTAAATACTGCAGCAAAGTTTTCAGCCACAAGGG
GAACCTGAACGTTCACCAGCGCACCCACTCCGGAGAGAAGCCCTACAAATGTCCCACC
TGTCAAAAGGCCTTCCGTCAGCTGGGGACATTCAAGCGTCACCTGAAAACACACCGGG
AAACCACCTCCCAGCTCGATGAGACGTAT
**SEQ ID NO:30 (pTriEx-1-ZSCAN5B [human])** 
GGAGTCGCTGCGACGCTGCCTTCGCCCCGTGCCCCGCTCCGCCGCCGCCTCGCGCCG
CCCGCCCCGGCTCTGACTGACCGCGTTACTCCCACAGGTGAGCGGGCGGGACGGCCC
TTCTCCTTCGGGCTGTAATTAGCGCTTGGTTTAATGACGGCTTGTTTCTTTTCTGTGGCT
GCGTGAAAGCCTTGAGGGGCTCCGGGAGGGCCCTTTGTGCGGGGGGAGCGGCTCGG
GGCTGTCCGCGGGGGGACGGCTGCCTTCGGGGGGGACGGGGCAGGGCGGGGTTCG
GCTTCTGGCGTGTGACCGGCGGCTCTAGAGCCTCTGCTAACCATGTTCATGCCTTCTTC
TTTTTCCTACAGCTCCTGGGCAACGTGCTGGTTATTGTGCTGTCTCATCATTTTGGCAAA
GAATTGGATCGGACCGAAATTAATACGACTCACTATAGGGGAATTGTGAGCGGATAACA
ATTCCCCGGAGTTAATCCGGGACCTTTAATTCAACCCAACACAATATATTATAGTTAAATA
AGAATTATTATCAAATCATTTGTATATTAATTAAAATACTATACTGTAAATTACATTTTATTT
ACAATCAAAGGAGATATACCATGGCTGCAAATTGGACACTCTCATGGGGTCAGGGAGGA
CCCTGCAACAGCCCTGGGTCAGACACTCCACGGTCTGTGGCGTCCCCAGAAACTCAAC
TTGGAAATCACGACAGGAACCCTGAGACTTGGCACATGAATTTCAGGATGTTCAGCTGC
CCAGAGGAGTCGGACCCCATCCAGGCTCTGAGGAAACTCACTGAGCTGTGCCATCTGT
GGCTGAGGCCCGACCTCCACACCAAAGAGCAGATCCTGGACATGCTGGTGATGGAGCA
GTTCATGATCTCCATGCCCCAGGAGCTCCAGGTCTTAGTCAAGGTGAACGGTGTGCAG
AGCTGCAAAGACCTGGAGGACCTGCTACGAAATAACAGAAGACCCAAGAAATGGTCTAT
AGTCAACTTGCTCGGCAAAGAATATCTTATGCTGAACTCAGATGTCGAGATGGCTGAAG
CCCCCGCCAGTGTCAGAGATGATCCGAGAGATGTGTCCAGCCAGTGGGCCTCCTCTGT
GAACCAGATGCATCCGGGGACAGGCCAGGCCCGCCGAGAGCAGCAGATCCTGCCCAG
 
GGTCGCTGCACTGTCCAGGAGGCAGGGAGAGGACTTTCTGCTACACAAGAGTATTGAC
GTAACAGGTGACCCAAACTCTCCGAGACCCAAGCAGACCTTGGAGAAGGATCTGAAGG
AAAACAGGGAAGAGAACCCAGGACTGTCATCCCCAGAGCCTCAGCTTCCAAAGAGTCC
CAATCTGGTGAGAGCAAAGGAGGGGAAGGAACCCCAGAAAAGAGCCTCTGTGGAAAAT
GTGGATGCTGACACACCTTCTGCCTGCGTTGTGGAGAGAGAAGCTTTGACTCACAGCG
GGAACAGAGGAGATGCTCTGAATCTGAGCAGTCCCAAAAGAAGCAAACCAGATGCCTC
CTCCATTTCCCAAGAAGAGCCTCAAGGAGAAGCCACACCTGTGGGCAACAGAGAATCC
CCAGGACAAGCTGAGATCAATCCAGTTCATTCCCCAGGCCCTGCGGGCCCGGTCAGTC
ACCCAGATGGCCAAGAAGCCAAGGCCCTGCCGCCCTTTGCATGTGACGTGTGCAATAA
ATCATTTAAGTATTTTTCCCAGCTAAGCATCCACAGGAGGTCACACACAGGAGACAGAC
CCTTTCAATGTGATCTCTGTCGGAAGCGCTTCTTGCAGCCCTCAGACCTCCGAGTTCAC
CAGCGAGTCCACACTGGCGAGAGGCCCTACATGTGTGACGTCTGCCAAAAGCGGTTCG
CCCACGAGTCCACCTTACAGGGCCACAAGAGGATCCACACCGGGGAGAGGCCGTTTAA
ATGTAAATACTGCAGCAAAGTTTTCAGCCACAAGGGGAACCTGAACGTTCACCAGCGCA
CCCACTCCGGAGAGAAGCCCTACAAATGTCCCACCTGTCAAAAGGCCTTCCGTCAGCT
GGGGACATTCAAGCGTCACCTGAAAACACACCGGGAAACCACCTCCCAGTGATAATCG
AGCACCACCATCACCATCACCATCACTAAGTGATTAACCTCAGGTGCAGGCTGCCTATC
AGAAGGTGGTGGCTGGTGTGGCCAATGCCCTGGCTCACAAATACCACTGAGATCGATC
TTTTTCCCTCTGCCAAAAATTATGGGGACATCATGAAGCCCCTTGAGCATCTGACTTCTG
GCTAATAAAGGAAATTTATTTTCATTGCAATAGTGTGTTGGAATTTTTTGTGTCTCTCACT
CGGAAGGACATATGGGAGGGCAAATCATTTAAAACATCAGAATGAGTATTTGGTTTAGA
GTTTGGCAACATATGCCCATATGTAACTAGCATAACCCCTTGGGGCCTCTAAACGGGTC
TTGAGGGGTTTTTTGCTGAAAGCATGCGGAGGAAATTCTCCTTGAAGTTTCCCTGGTGT
TCAAAGTAAAGGAGTTTGCACCAGACGCACCTCTGTTCACTGGTCCGGCGTATTAAAAC
ACGATACATTGTTATTAGTACATTTATTAAGCGCTAGATTCTGTGCGTTGTTGATTTACAG
ACAATTGTTGTACGTATTTTAATAATTCATTAAATTTATAATCTTTAGGGTGGTATGTTAGA
GCGAAAATCAAATGATTTTCAGCGTCTTTATATCTGAATTTAAATATTAAATCCTCAATAG
ATTTGTAAAATAGGTTTCGATTAGTTTCAAACAAGGGTTGTTTTTCCGAACCGATGGCTG
GACTATCTAATGGATTTTCGCTCAACGCCACAAAACTTGCCAAATCTTGTAGCAGCAATC
TAGCTTTGTCGATATTCGTTTGTGTTTTGTTTTGTAATAAAGGTTCGACGTCGTTCAAAAT
ATTATGCGCTTTTGTATTTCTTTCATCACTGTCGTTAGTGTACAATTGACTCGACGTAAAC
ACGTTAAATAGAGCTTGGACATATTTAACATCGGGCGTGTTAGCTTTATTAGGCCGATTA
TCGTCGTCGTCCCAACCCTCGTCGTTAGAAGTTGCTTCCGAAGACGATTTTGCCATAGC
CACACGACGCCTATTAATTGTGTCGGCTAACACGTCCGCGATCAAATTTGTAGTTGAGC
TTTTTGGAATTATTTCTGATTGCGGGCGTTTTTGGGCGGGTTTCAATCTAACTGTGCCCG
ATTTTAATTCAGACAACACGTTAGAAAGCGATGGTGCAGGCGGTGGTAACATTTCAGAC
GGCAAATCTACTAATGGCGGCGGTGGTGGAGCTGATGATAAATCTACCATCGGTGGAG
GCGCAGGCGGGGCTGGCGGCGGAGGCGGAGGCGGAGGTGGTGGCGGTGATGCAGA
CGGCGGTTTAGGCTCAAATGTCTCTTTAGGCAACACAGTCGGCACCTCAACTATTGTAC
TGGTTTCGGGCGCCGTTTTTGGTTTGACCGGTCTGAGACGAGTGCGATTTTTTTCGTTT
CTAATAGCTTCCAACAATTGTTGTCTGTCGTCTAAAGGTGCAGCGGGTTGAGGTTCCGT
CGGCATTGGTGGAGCGGGCGGCAATTCAGACATCGATGGTGGTGGTGGTGGTGGAGG
CGCTGGAATGTTAGGCACGGGAGAAGGTGGTGGCGGCGGTGCCGCCGGTATAATTTG
TTCTGGTTTAGTTTGTTCGCGCACGATTGTGGGCACCGGCGCAGGCGCCGCTGGCTGC
ACAACGGAAGGTCGTCTGCTTCGAGGCAGCGCTTGGGGTGGTGGCAATTCAATATTATA
ATTGGAATACAAATCGTAAAAATCTGCTATAAGCATTGTAATTTCGCTATCGTTTACCGTG
CCGATATTTAACAACCGCTCAATGTAAGCAATTGTATTGTAAAGAGATTGTCTCAAGCTC
 
GGAACGCTGCGCTCGGTCGTTCGGCTGCGGCGAGCGGTATCAGCTCACTCAAAGGCG
GTAATACGGTTATCCACAGAATCAGGGGATAACGCAGGAAAGAACATGTGAGCAAAAGG
CCAGCAAAAGGCCAGGAACCGTAAAAAGGCCGCGTTGCTGGCGTTTTTCCATAGGCTC
CGCCCCCCTGACGAGCATCACAAAAATCGACGCTCAAGTCAGAGGTGGCGAAACCCGA
CAGGACTATAAAGATACCAGGCGTTTCCCCCTGGAAGCTCCCTCGTGCGCTCTCCTGTT
CCGACCCTGCCGCTTACCGGATACCTGTCCGCCTTTCTCCCTTCGGGAAGCGTGGCGC
TTTCTCAATGCTCACGCTGTAGGTATCTCAGTTCGGTGTAGGTCGTTCGCTCCAAGCTG
GGCTGTGTGCACGAACCCCCCGTTCAGCCCGACCGCTGCGCCTTATCCGGTAACTATC
GTCTTGAGTCCAACCCGGTAAGACACGACTTATCGCCACTGGCAGCAGCCACTGGTAA
CAGGATTAGCAGAGCGAGGTATGTAGGCGGTGCTACAGAGTTCTTGAAGTGGTGGCCT
AACTACGGCTACACTAGAAGGACAGTATTTGGTATCTGCGCTCTGCTGAAGCCAGTTAC
CTTCGGAAAAAGAGTTGGTAGCTCTTGATCCGGCAAACAAACCACCGCTGGTAGCGGT
GGTTTTTTTGTTTGCAAGCAGCAGATTACGCGCAGAAAAAAAGGATCTCAAGAAGATCCT
TTGTTACCAATGCTTAATCAGTGAGGCACCTATCTCAGCGATCTGTCTATTTCGTTCATC
CATAGTTGCCTGACTCCCCGTCGTGTAGATAACTACGATACGGGAGGGCTTACCATCTG
GCCCCAGTGCTGCAATGATACCGCGAGACCCACGCTCACCGGCTCCAGATTTATCAGC
AATAAACCAGCCAGCCGGAAGGGCCGAGCGCAGAAGTGGTCCTGCAACTTTATCCGCC
TCCATCCAGTCTATTAATTGTTGCCGGGAAGCTAGAGTAAGTAGTTCGCCAGTTAATAGT
TTGCGCAACGTTGTTGCCATTGCTACAGGCATCGTGGTGTCACGCTCGTCGTTTGGTAT
GGCTTCATTCAGCTCCGGTTCCCAACGATCAAGGCGAGTTACATGATCCCCCATGTTGT
GCAAAAAAGCGGTTAGCTCCTTCGGTCCTCCGATCGTTGTCAGAAGTAAGTTGGCCGCA
GTGTTATCACTCATGGTTATGGCAGCACTGCATAATTCTCTTACTGTCATGCCATCCGTA
AGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGA
ACTTTAAAAGTGCTCATCATTGGAAAACGTTCTTCGGGGCGAAAACTCTCAAGGATCTTA
CCGCTGTTGAGATCCAGTTCGATGTAACCCACTCGTGCACCCAACTGATCTTCAGCATC
TTTTACTTTCACCAGCGTTTCTGGGTGAGCAAAAACAGGAAGGCAAAATGCCGCAAAAA
AGGGAATAAGGGCGACACGGAAATGTTGAATACTCATACTCTTCCTTTTTCAATATTATT
GAAGCATTTATCAGGGTTATTGTCTCATGTCCGCGCGTTTCCTGCATCTTTTAATCAAAT
CCCAAGATGTGTATAAACCACCAAACTGCCAAAAAATGAAAACTGTCGACAAGCTCTGT
CCGTTTGCTGGCAACTGCAAGGGTCTCAATCCTATTTGTAATTATTGAATAATAAAACAA
TTATAAATGTCAAATTTGTTTTTTATTAACGATACAAACCAAACGCAACAAGAACATTTGT
AGTATTATCTATAATTGAAAACGCGTAGTTATAATCGCTGAGGTAATATTTAAAATCATTT
TCAAATGATTCACAGTTAATTTGCGACAATATAATTTTATTTTCACATAAACTAGACGCCT
TGTCGTCTTCTTCTTCGTATTCCTTCTCTTTTTCATTTTTCTCTTCATAAAAATTAACATAG
TTATTATCGTATCCATATATGTATCTATCGTATAGAGTAAATTTTTTGTTGTCATAAATATA
TATGTCTTTTTTAATGGGGTGTATAGTACCGCTGCGCATAGTTTTTCTGTAATTTACAACA
GTGCTATTTTCTGGTAGTTCTTCGGAGTGTGTTGCTTTAATTATTAAATTTATATAATCAAT
GAATTTGGGATCGTCGGTTTTGTACAATATGTTGCCGGCATAGTACGCAGCTTCTTCTAG
TTCAATTACACCATTTTTTAGCAGCACCGGATTAACATAACTTTCCAAAATGTTGTACGAA
CCGTTAAACAAAAACAGTTCACCTCCCTTTTCTATACTATTGTCTGCGAGCAGTTGTTTG
TTGTTAAAAATAACAGCCATTGTAATGAGACGCACAAACTAATATCACAAACTGGAAATG
TCTATCAATATATAGTTGCTCTAGTTATTAATAGTAATCAATTACGGGGTCATTAGTTCAT
AGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAA
TAGGGACTTTCCATTGACGTCAATGGGTGGACTATTTACGGTAAACTGCCCACTTGGCA
GTACATCAAGTGTATCATATGCCAAGTACGCCCCCTATTGACGTCAATGACGGTAAATG
 
GCCCGCCTGGCATTATGCCCAGTACATGACCTTATGGGACTTTCCTACTTGGCAGTACA
TCTACGTATTAGTCATCGCTATTACCATGCATGGTCGAGGTGAGCCCCACGTTCTGCTT
CACTCTCCCCATCTCCCCCCCCTCCCCACCCCCAATTTTGTATTTATTTATTTTTTAATTA
TTTTGTGCAGCGATGGGGGCGGGGGGGGGGGGGGGGCGCGCGCCAGGCGGGGCGG
GGCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCTA
TAAAAAGCGAAGCGCGCGGCGGGCG
**SEQ ID NO:31 (ZSCAN5B[human])** 
MAANWTLSWGQGGPCNSPGSDTPRSVASPETQLGNHDRNPETWHMNFRMFSCPEESDP
IQALRKLTELCHLWLRPDLHTKEQILDMLVMEQFMISMPQELQVLVKVNGVQSCKDLEDLLR
NNRRPKKWSIVNLLGKEYLMLNSDVEMAEAPASVRDDPRDVSSQWASSVNQMHPGTGQA
RREQQILPRVAALSRRQGEDFLLHKSIDVTGDPNSPRPKQTLEKDLKENREENPGLSSPEP
QLPKSPNLVRAKEGKEPQKRASVENVDADTPSACVVEREALTHSGNRGDALNLSSPKRSK
PDASSISQEEPQGEATPVGNRESPGQAEINPVHSPGPAGPVSHPDGQEAKALPPFACDVC
NKSFKYFSQLSIHRRSHTGDRPFQCDLCRKRFLQPSDLRVHQRVHTGERPYMCDVCQKRF
AHESTLQGHKRIHTGERPFKCKYCSKVFSHKGNLNVHQRTHSGEKPYKCPTCQKAFRQLG
TFKRHLKTHRETTSQ
**SEQ ID NO:32 (pTriEx-1-ZSCAN5B[human]-His)** 
GGAGTCGCTGCGACGCTGCCTTCGCCCCGTGCCCCGCTCCGCCGCCGCCTCGCGCCG
CCCGCCCCGGCTCTGACTGACCGCGTTACTCCCACAGGTGAGCGGGCGGGACGGCCC
TTCTCCTTCGGGCTGTAATTAGCGCTTGGTTTAATGACGGCTTGTTTCTTTTCTGTGGCT
GCGTGAAAGCCTTGAGGGGCTCCGGGAGGGCCCTTTGTGCGGGGGGAGCGGCTCGG
GGCTGTCCGCGGGGGGACGGCTGCCTTCGGGGGGGACGGGGCAGGGCGGGGTTCG
GCTTCTGGCGTGTGACCGGCGGCTCTAGAGCCTCTGCTAACCATGTTCATGCCTTCTTC
TTTTTCCTACAGCTCCTGGGCAACGTGCTGGTTATTGTGCTGTCTCATCATTTTGGCAAA
GAATTGGATCGGACCGAAATTAATACGACTCACTATAGGGGAATTGTGAGCGGATAACA
ATTCCCCGGAGTTAATCCGGGACCTTTAATTCAACCCAACACAATATATTATAGTTAAATA
AGAATTATTATCAAATCATTTGTATATTAATTAAAATACTATACTGTAAATTACATTTTATTT
ACAATCAAAGGAGATATACCATGGCTGCAAATTGGACACTCTCATGGGGTCAGGGAGGA
CCCTGCAACAGCCCTGGGTCAGACACTCCACGGTCTGTGGCGTCCCCAGAAACTCAAC
TTGGAAATCACGACAGGAACCCTGAGACTTGGCACATGAATTTCAGGATGTTCAGCTGC
CCAGAGGAGTCGGACCCCATCCAGGCTCTGAGGAAACTCACTGAGCTGTGCCATCTGT
GGCTGAGGCCCGACCTCCACACCAAAGAGCAGATCCTGGACATGCTGGTGATGGAGCA
GTTCATGATCTCCATGCCCCAGGAGCTCCAGGTCTTAGTCAAGGTGAACGGTGTGCAG
AGCTGCAAAGACCTGGAGGACCTGCTACGAAATAACAGAAGACCCAAGAAATGGTCTAT
AGTCAACTTGCTCGGCAAAGAATATCTTATGCTGAACTCAGATGTCGAGATGGCTGAAG
CCCCCGCCAGTGTCAGAGATGATCCGAGAGATGTGTCCAGCCAGTGGGCCTCCTCTGT
GAACCAGATGCATCCGGGGACAGGCCAGGCCCGCCGAGAGCAGCAGATCCTGCCCAG
GGTCGCTGCACTGTCCAGGAGGCAGGGAGAGGACTTTCTGCTACACAAGAGTATTGAC
GTAACAGGTGACCCAAACTCTCCGAGACCCAAGCAGACCTTGGAGAAGGATCTGAAGG
AAAACAGGGAAGAGAACCCAGGACTGTCATCCCCAGAGCCTCAGCTTCCAAAGAGTCC
CAATCTGGTGAGAGCAAAGGAGGGGAAGGAACCCCAGAAAAGAGCCTCTGTGGAAAAT
GTGGATGCTGACACACCTTCTGCCTGCGTTGTGGAGAGAGAAGCTTTGACTCACAGCG
GGAACAGAGGAGATGCTCTGAATCTGAGCAGTCCCAAAAGAAGCAAACCAGATGCCTC
CTCCATTTCCCAAGAAGAGCCTCAAGGAGAAGCCACACCTGTGGGCAACAGAGAATCC
CCAGGACAAGCTGAGATCAATCCAGTTCATTCCCCAGGCCCTGCGGGCCCGGTCAGTC
 
ACCCAGATGGCCAAGAAGCCAAGGCCCTGCCGCCCTTTGCATGTGACGTGTGCAATAA
ATCATTTAAGTATTTTTCCCAGCTAAGCATCCACAGGAGGTCACACACAGGAGACAGAC
CCTTTCAATGTGATCTCTGTCGGAAGCGCTTCTTGCAGCCCTCAGACCTCCGAGTTCAC
CAGCGAGTCCACACTGGCGAGAGGCCCTACATGTGTGACGTCTGCCAAAAGCGGTTCG
CCCACGAGTCCACCTTACAGGGCCACAAGAGGATCCACACCGGGGAGAGGCCGTTTAA
ATGTAAATACTGCAGCAAAGTTTTCAGCCACAAGGGGAACCTGAACGTTCACCAGCGCA
CCCACTCCGGAGAGAAGCCCTACAAATGTCCCACCTGTCAAAAGGCCTTCCGTCAGCT
GGGGACATTCAAGCGTCACCTGAAAACACACCGGGAAACCACCTCCCAGCTCGAGCAC
CACCATCACCATCACCATCACTAAGTGATTAACCTCAGGTGCAGGCTGCCTATCAGAAG
GTGGTGGCTGGTGTGGCCAATGCCCTGGCTCACAAATACCACTGAGATCGATCTTTTTC
CCTCTGCCAAAAATTATGGGGACATCATGAAGCCCCTTGAGCATCTGACTTCTGGCTAA
TAAAGGAAATTTATTTTCATTGCAATAGTGTGTTGGAATTTTTTGTGTCTCTCACTCGGAA
GGACATATGGGAGGGCAAATCATTTAAAACATCAGAATGAGTATTTGGTTTAGAGTTTGG
CAACATATGCCCATATGTAACTAGCATAACCCCTTGGGGCCTCTAAACGGGTCTTGAGG
GGTTTTTTGCTGAAAGCATGCGGAGGAAATTCTCCTTGAAGTTTCCCTGGTGTTCAAAGT
AAAGGAGTTTGCACCAGACGCACCTCTGTTCACTGGTCCGGCGTATTAAAACACGATAC
ATTGTTATTAGTACATTTATTAAGCGCTAGATTCTGTGCGTTGTTGATTTACAGACAATTG
TTGTACGTATTTTAATAATTCATTAAATTTATAATCTTTAGGGTGGTATGTTAGAGCGAAA
ATCAAATGATTTTCAGCGTCTTTATATCTGAATTTAAATATTAAATCCTCAATAGATTTGTA
AAATAGGTTTCGATTAGTTTCAAACAAGGGTTGTTTTTCCGAACCGATGGCTGGACTATC
TAATGGATTTTCGCTCAACGCCACAAAACTTGCCAAATCTTGTAGCAGCAATCTAGCTTT
GTCGATATTCGTTTGTGTTTTGTTTTGTAATAAAGGTTCGACGTCGTTCAAAATATTATGC
GCTTTTGTATTTCTTTCATCACTGTCGTTAGTGTACAATTGACTCGACGTAAACACGTTAA
ATAGAGCTTGGACATATTTAACATCGGGCGTGTTAGCTTTATTAGGCCGATTATCGTCGT
CGTCCCAACCCTCGTCGTTAGAAGTTGCTTCCGAAGACGATTTTGCCATAGCCACACGA
CGCCTATTAATTGTGTCGGCTAACACGTCCGCGATCAAATTTGTAGTTGAGCTTTTTGGA
ATTATTTCTGATTGCGGGCGTTTTTGGGCGGGTTTCAATCTAACTGTGCCCGATTTTAAT
TCAGACAACACGTTAGAAAGCGATGGTGCAGGCGGTGGTAACATTTCAGACGGCAAAT
CTACTAATGGCGGCGGTGGTGGAGCTGATGATAAATCTACCATCGGTGGAGGCGCAGG
CGGGGCTGGCGGCGGAGGCGGAGGCGGAGGTGGTGGCGGTGATGCAGACGGCGGTT
TAGGCTCAAATGTCTCTTTAGGCAACACAGTCGGCACCTCAACTATTGTACTGGTTTCG
GGCGCCGTTTTTGGTTTGACCGGTCTGAGACGAGTGCGATTTTTTTCGTTTCTAATAGCT
TCCAACAATTGTTGTCTGTCGTCTAAAGGTGCAGCGGGTTGAGGTTCCGTCGGCATTGG
TGGAGCGGGCGGCAATTCAGACATCGATGGTGGTGGTGGTGGTGGAGGCGCTGGAAT
GTTAGGCACGGGAGAAGGTGGTGGCGGCGGTGCCGCCGGTATAATTTGTTCTGGTTTA
GTTTGTTCGCGCACGATTGTGGGCACCGGCGCAGGCGCCGCTGGCTGCACAACGGAA
GGTCGTCTGCTTCGAGGCAGCGCTTGGGGTGGTGGCAATTCAATATTATAATTGGAATA
CAAATCGTAAAAATCTGCTATAAGCATTGTAATTTCGCTATCGTTTACCGTGCCGATATTT
AACAACCGCTCAATGTAAGCAATTGTATTGTAAAGAGATTGTCTCAAGCTCGGAACGCT
GCGCTCGGTCGTTCGGCTGCGGCGAGCGGTATCAGCTCACTCAAAGGCGGTAATACG
GTTATCCACAGAATCAGGGGATAACGCAGGAAAGAACATGTGAGCAAAAGGCCAGCAA
AAGGCCAGGAACCGTAAAAAGGCCGCGTTGCTGGCGTTTTTCCATAGGCTCCGCCCCC
CTGACGAGCATCACAAAAATCGACGCTCAAGTCAGAGGTGGCGAAACCCGACAGGACT
ATAAAGATACCAGGCGTTTCCCCCTGGAAGCTCCCTCGTGCGCTCTCCTGTTCCGACCC
TGCCGCTTACCGGATACCTGTCCGCCTTTCTCCCTTCGGGAAGCGTGGCGCTTTCTCAA
TGCTCACGCTGTAGGTATCTCAGTTCGGTGTAGGTCGTTCGCTCCAAGCTGGGCTGTGT
GCACGAACCCCCCGTTCAGCCCGACCGCTGCGCCTTATCCGGTAACTATCGTCTTGAG
 
TCCAACCCGGTAAGACACGACTTATCGCCACTGGCAGCAGCCACTGGTAACAGGATTA
GCAGAGCGAGGTATGTAGGCGGTGCTACAGAGTTCTTGAAGTGGTGGCCTAACTACGG
CTACACTAGAAGGACAGTATTTGGTATCTGCGCTCTGCTGAAGCCAGTTACCTTCGGAA
AAAGAGTTGGTAGCTCTTGATCCGGCAAACAAACCACCGCTGGTAGCGGTGGTTTTTTT
GTTTGCAAGCAGCAGATTACGCGCAGAAAAAAAGGATCTCAAGAAGATCCTTTGTTACC
AATGCTTAATCAGTGAGGCACCTATCTCAGCGATCTGTCTATTTCGTTCATCCATAGTTG
CCTGACTCCCCGTCGTGTAGATAACTACGATACGGGAGGGCTTACCATCTGGCCCCAG
TGCTGCAATGATACCGCGAGACCCACGCTCACCGGCTCCAGATTTATCAGCAATAAACC
AGCCAGCCGGAAGGGCCGAGCGCAGAAGTGGTCCTGCAACTTTATCCGCCTCCATCCA
GTCTATTAATTGTTGCCGGGAAGCTAGAGTAAGTAGTTCGCCAGTTAATAGTTTGCGCAA
CGTTGTTGCCATTGCTACAGGCATCGTGGTGTCACGCTCGTCGTTTGGTATGGCTTCAT
TCAGCTCCGGTTCCCAACGATCAAGGCGAGTTACATGATCCCCCATGTTGTGCAAAAAA
GCGGTTAGCTCCTTCGGTCCTCCGATCGTTGTCAGAAGTAAGTTGGCCGCAGTGTTATC
ACTCATGGTTATGGCAGCACTGCATAATTCTCTTACTGTCATGCCATCCGTAAGATGCTT
TTCTGTGACTGGTGAGTACTCAACCAAGTCATTCTGAGAATAGTGTATGCGGCGACCGA
GTTGCTCTTGCCCGGCGTCAATACGGGATAATACCGCGCCACATAGCAGAACTTTAAAA
GTGCTCATCATTGGAAAACGTTCTTCGGGGCGAAAACTCTCAAGGATCTTACCGCTGTT
GAGATCCAGTTCGATGTAACCCACTCGTGCACCCAACTGATCTTCAGCATCTTTTACTTT
CACCAGCGTTTCTGGGTGAGCAAAAACAGGAAGGCAAAATGCCGCAAAAAAGGGAATA
AGGGCGACACGGAAATGTTGAATACTCATACTCTTCCTTTTTCAATATTATTGAAGCATTT
ATCAGGGTTATTGTCTCATGTCCGCGCGTTTCCTGCATCTTTTAATCAAATCCCAAGATG
TGTATAAACCACCAAACTGCCAAAAAATGAAAACTGTCGACAAGCTCTGTCCGTTTGCTG
GCAACTGCAAGGGTCTCAATCCTATTTGTAATTATTGAATAATAAAACAATTATAAATGTC
AAATTTGTTTTTTATTAACGATACAAACCAAACGCAACAAGAACATTTGTAGTATTATCTA
TAATTGAAAACGCGTAGTTATAATCGCTGAGGTAATATTTAAAATCATTTTCAAATGATTC
ACAGTTAATTTGCGACAATATAATTTTATTTTCACATAAACTAGACGCCTTGTCGTCTTCT
TCTTCGTATTCCTTCTCTTTTTCATTTTTCTCTTCATAAAAATTAACATAGTTATTATCGTAT
CCATATATGTATCTATCGTATAGAGTAAATTTTTTGTTGTCATAAATATATATGTCTTTTTT
AATGGGGTGTATAGTACCGCTGCGCATAGTTTTTCTGTAATTTACAACAGTGCTATTTTC
TGGTAGTTCTTCGGAGTGTGTTGCTTTAATTATTAAATTTATATAATCAATGAATTTGGGA
TCGTCGGTTTTGTACAATATGTTGCCGGCATAGTACGCAGCTTCTTCTAGTTCAATTACA
CCATTTTTTAGCAGCACCGGATTAACATAACTTTCCAAAATGTTGTACGAACCGTTAAAC
AAAAACAGTTCACCTCCCTTTTCTATACTATTGTCTGCGAGCAGTTGTTTGTTGTTAAAAA
TAACAGCCATTGTAATGAGACGCACAAACTAATATCACAAACTGGAAATGTCTATCAATA
TATAGTTGCTCTAGTTATTAATAGTAATCAATTACGGGGTCATTAGTTCATAGCCCATATA
TGGAGTTCCGCGTTACATAACTTACGGTAAATGGCCCGCCTGGCTGACCGCCCAACGA
CCCCCGCCCATTGACGTCAATAATGACGTATGTTCCCATAGTAACGCCAATAGGGACTT
TCCATTGACGTCAATGGGTGGACTATTTACGGTAAACTGCCCACTTGGCAGTACATCAA
GTGTATCATATGCCAAGTACGCCCCCTATTGACGTCAATGACGGTAAATGGCCCGCCTG
GCATTATGCCCAGTACATGACCTTATGGGACTTTCCTACTTGGCAGTACATCTACGTATT
AGTCATCGCTATTACCATGCATGGTCGAGGTGAGCCCCACGTTCTGCTTCACTCTCCCC
ATCTCCCCCCCCTCCCCACCCCCAATTTTGTATTTATTTATTTTTTAATTATTTTGTGCAG
CGATGGGGGCGGGGGGGGGGGGGGGGCGCGCGCCAGGCGGGGCGGGGCGGGGCG
AGGGGCGGGGCGGGGCGAGGCGGAGAGGTGCGGCGGCAGCCAATCAGAGCGGCGC
GCTCCGAAAGTTTCCTTTTATGGCGAGGCGGCGGCGGCGGCGGCCCTATAAAAAGCGA
AGCGCGCGGCGGGCG
**SEQ** ID **NO:33 (VGLUT2[human]-H8)** 
MAANWTLSWGQGGPCNSPGSDTPRSVASPETQLGNHDRNPETWHMNFRMFSCPEESDP
IQALRKLTELCHLWLRPDLHTKEQILDMLVMEQFMISMPQELQVLVKVNGVQSCKDLEDLLR
NNRRPKKWSIVNLLGKEYLMLNSDVEMAEAPASVRDDPRDVSSQWASSVNQMHPGTGQA
RREQQILPRVAALSRRQGEDFLLHKSIDVTGDPNSPRPKQTLEKDLKENREENPGLSSPEP
QLPKSPNLVRAKEGKEPQKRASVENVDADTPSACVVEREALTHSGNRGDALNLSSPKRSK
PDASSISQEEPQGEATPVGNRESPGQAEINPVHSPGPAGPVSHPDGQEAKALPPFACDVC
NKSFKYFSQLSIHRRSHTGDRPFQCDLCRKRFLQPSDLRVHQRVHTGERPYMCDVCQKRF
AHESTLQGHKRIHTGERPFKCKYCSKVFSHKGNLNVHQRTHSGEKPYKCPTCQKAFRQLG
TFKRHLKTHRETTSQLEHHHHHHHH
**SEQ ID NO:34 (GamS-ZSCAN5A-6His)** 
MGNAYYIQDRLEAQSWARHYQQLAREEKEAELADDMEKGLPQHLFESLCIDHLQRHGASK
KSITRAFDDDVEFQERMAEHIRYMVETIAHHQVDIDSEVGGSGTPSACVVEREASTHSGSR
GDALNLRGLKRSKPDATSISQEEPQGEATPVGNRESPGQAEINPVHSPGPAGPVSHPSGQ
EVKELLPFACEVGGSGHHHHHH
**SEQ ID NO:35 (GamS-ZSCAN5B-6His)** 
MGNAYYIQDRLEAQSWARHYQQLAREEKEAELADDMEKGLPQHLFESLCIDHLQRHGASK
KSITRAFDDDVEFQERMAEHIRYMVETIAHHQVDIDSEVGGSGSACVVEREALTHSGNRGD
ALNLSSPKRSKPDASSISQEEPQGEATPVGNRESPGQAEINPVHSPGPAGPVSHPDGQEA
KALPPFACDVGGSGHHHHHH
**SEQ ID NO:36 (GamS-ZSCAN5C-6His)** 
MGNAYYIQDRLEAQSWARHYQQLAREEKEAELADDMEKGLPQHLFESLCIDHLQRHGASK
KSITRAFDDDVEFQERMAEHIRYMVETIAHHQVDIDSEVGGSGTPSACVVEREASTHSGNR
GDALNLSSPKRSKPDASSISQEEPQGEATPVGNRESPGQAGMNSIHSPGPASPVSHPDGQ
EAKALPPFACDVGGSGHHHHHH
**SEQ ID NO:37 (GamS-ZSCAN5A-6His gene fragment with adapters)** 
GAGGTGACGGGCCTCAAAGCCGCGTGGGAGTGAGCTAACACCGTGCGTGTTGACAATT
TTACCTCTGGCGGTGATAATGGTTGCAGCTACTAATAATTTTGTTTAACTTTAAGAAGGA
GATATACCATGGGAAACGCTTATTACATTCAGGATCGTCTTGAGGCTCAGAGCTGGGCG
CGTCACTACCAGCAGCTCGCCCGTGAAGAGAAAGAGGCAGAACTGGCAGACGACATGG
AAAAAGGCCTGCCCCAGCACCTGTTTGAATCGCTATGCATCGATCATTTGCAACGCCAC
GGGGCCAGCAAAAAATCCATTACCCGTGCGTTTGATGACGATGTTGAGTTTCAGGAGCG
CATGGCAGAACACATCCGGTACATGGTTGAAACCATTGCTCACCACCAGGTTGATATTG
ATTCAGAGGTAGGTGGTAGCGGTACTCCGAGCGCATGTGTGGTGGAACGAGAAGCATC
GACACATTCAGGGAGCAGAGGGGACGCCCTGAATTTACGTGGCCTCAAACGTTCCAAA
CCCGATGCTACTTCTATTAGCCAAGAGGAACCGCAAGGAGAGGCAACACCGGTAGGCA
ACCGTGAATCTCCTGGCCAGGCAGAGATCAACCCTGTTCACTCCCCTGGTCCCGCTGG
TCCGGTTTCTCATCCGAGCGGGCAGGAGGTCAAAGAACTTCTTCCATTTGCGTGTGAAG
TAGGTGGTAGCGGTCATCATCACCATCATCATTAAAATGCAAAGCCCGCCGAAAGGCGG
GCTTTTCTGTACTTCGCAACGATTTCGGAGTCCGGAGACTCGCTGTTTTCGAAATTTGC
GCTCAAGGGCGGGTATTGAACC,843,CAATCCGCCCTCACTACAACCGGAGGTGACGG
GCCTCAAAGCCGCGTGGGAGTGAGCTAACACCGTGCGTGTTGACAATTTTACCTCTGG
CGGTGATAATGGTTGCAGCTACTAATAATTTTGTTTAACTTTAAGAAGGAGATATACCAT
GGGAAACGCTTATTACATTCAGGATCGTCTTGAGGCTCAGAGCTGGGCGCGTCACTAC
CAGCAGCTCGCCCGTGAAGAGAAAGAGGCAGAACTGGCAGACGACATGGAAAAAGGC
 
CTGCCCCAGCACCTGTTTGAATCGCTATGCATCGATCATTTGCAACGCCACGGGGCCA
GCAAAAAATCCATTACCCGTGCGTTTGATGACGATGTTGAGTTTCAGGAGCGCATGGCA
GAACACATCCGGTACATGGTTGAAACCATTGCTCACCACCAGGTTGATATTGATTCAGA
GGTAGGTGGTAGCGGTACTCCGAGCGCATGTGTGGTGGAACGAGAAGCATCGACACAT
TCAGGGAGCAGAGGGGACGCCCTGAATTTACGTGGCCTCAAACGTTCCAAACCCGATG
CTACTTCTATTAGCCAAGAGGAACCGCAAGGAGAGGCAACACCGGTAGGCAACCGTGA
ATCTCCTGGCCAGGCAGAGATCAACCCTGTTCACTCCCCTGGTCCCGCTGGTCCGGTT
TCTCATCCGAGCGGGCAGGAGGTCAAAGAACTTCTTCCATTTGCGTGTGAAGTAGGTG
GTAGCGGTCATCATCACCATCATCATTAAAATGCAAAGCCCGCCGAAAGGCGGGCTTTT
CTGTACTTCGCAACGATTTCGGAGTCCGGAGACTCGCTGTTTTCGAAATTTGCGCTCAA
GGGCGGGTATTGAACCCTACTCTGGCGTCGATGAGGGA
SEQ ID NO:38 (GamS-ZSCAN5B-6His gene fragment with adapters) 
GAGGTGACGGGCCTCAAAGCCGCGTGGGAGTGAGCTAACACCGTGCGTGTTGACAATT
TTACCTCTGGCGGTGATAATGGTTGCAGCTACTAATAATTTTGTTTAACTTTAAGAAGGA
GATATACCATGGGAAACGCTTATTACATTCAGGATCGTCTTGAGGCTCAGAGCTGGGCG
CGTCACTACCAGCAGCTCGCCCGTGAAGAGAAAGAGGCAGAACTGGCAGACGACATGG
AAAAAGGCCTGCCCCAGCACCTGTTTGAATCGCTATGCATCGATCATTTGCAACGCCAC
GGGGCCAGCAAAAAATCCATTACCCGTGCGTTTGATGACGATGTTGAGTTTCAGGAGCG
CATGGCAGAACACATCCGGTACATGGTTGAAACCATTGCTCACCACCAGGTTGATATTG
ATTCAGAGGTAGGTGGTAGCGGTTCTGCTTGTGTTGTGGAACGGGAAGCGCTGACTCA
TAGTGGCAATCGCGGTGACGCACTCAATCTTAGCAGCCCCAAACGCAGCAAGCCGGAC
GCGTCTAGCATTAGCCAGGAAGAACCGCAGGGAGAAGCAACTCCTGTTGGCAACCGAG
AATCCCCTGGGCAAGCCGAAATCAATCCTGTACACTCTCCGGGACCTGCAGGCCCCGT
TTCACATCCGGATGGACAAGAGGCGAAAGCACTCCCGCCGTTCGCATGCGATGTGGGT
GGTAGCGGTCATCATCACCATCATCATTAAAATGCAAAGCCCGCCGAAAGGCGGGCTTT
TCTGTACTTCGCAACGATTTCGGAGTCCGGAGACTCGCTGTTTTCGAAATTTGCGCTCA
AGGGCGGGTATTGAACC,837,CAATCCGCCCTCACTACAACCGGAGGTGACGGGCCTCA
AAGCCGCGTGGGAGTGAGCTAACACCGTGCGTGTTGACAATTTTACCTCTGGCGGTGA
TAATGGTTGCAGCTACTAATAATTTTGTTTAACTTTAAGAAGGAGATATACCATGGGAAA
CGCTTATTACATTCAGGATCGTCTTGAGGCTCAGAGCTGGGCGCGTCACTACCAGCAG
CTCGCCCGTGAAGAGAAAGAGGCAGAACTGGCAGACGACATGGAAAAAGGCCTGCCC
CAGCACCTGTTTGAATCGCTATGCATCGATCATTTGCAACGCCACGGGGCCAGCAAAAA
ATCCATTACCCGTGCGTTTGATGACGATGTTGAGTTTCAGGAGCGCATGGCAGAACACA
TCCGGTACATGGTTGAAACCATTGCTCACCACCAGGTTGATATTGATTCAGAGGTAGGT
GGTAGCGGTTCTGCTTGTGTTGTGGAACGGGAAGCGCTGACTCATAGTGGCAATCGCG
GTGACGCACTCAATCTTAGCAGCCCCAAACGCAGCAAGCCGGACGCGTCTAGCATTAG
CCAGGAAGAACCGCAGGGAGAAGCAACTCCTGTTGGCAACCGAGAATCCCCTGGGCAA
GCCGAAATCAATCCTGTACACTCTCCGGGACCTGCAGGCCCCGTTTCACATCCGGATG
GACAAGAGGCGAAAGCACTCCCGCCGTTCGCATGCGATGTGGGTGGTAGCGGTCATCA
TCACCATCATCATTAAAATGCAAAGCCCGCCGAAAGGCGGGCTTTTCTGTACTTCGCAA
CGATTTCGGAGTCCGGAGACTCGCTGTTTTCGAAATTTGCGCTCAAGGGCGGGTATTGA
ACCCTACTCTGGCGTCGATGAGGGA
SEQ ID NO:39 (GamS-ZSCAN5C-6His gene fragment with adapters) 
GAGGTGACGGGCCTCAAAGCCGCGTGGGAGTGAGCTAACACCGTGCGTGTTGACAATT
TTACCTCTGGCGGTGATAATGGTTGCAGCTACTAATAATTTTGTTTAACTTTAAGAAGGA
GATATACCATGGGAAACGCTTATTACATTCAGGATCGTCTTGAGGCTCAGAGCTGGGCG
 
CGTCACTACCAGCAGCTCGCCCGTGAAGAGAAAGAGGCAGAACTGGCAGACGACATGG
AAAAAGGCCTGCCCCAGCACCTGTTTGAATCGCTATGCATCGATCATTTGCAACGCCAC
GGGGCCAGCAAAAAATCCATTACCCGTGCGTTTGATGACGATGTTGAGTTTCAGGAGCG
CATGGCAGAACACATCCGGTACATGGTTGAAACCATTGCTCACCACCAGGTTGATATTG
ATTCAGAGGTAGGTGGTAGCGGTACCCCAAGCGCTTGTGTAGTCGAAAGAGAAGCAAG
TACGCACAGCGGCAATCGGGGGGACGCCTTGAACTTGTCTTCTCCTAAAAGATCGAAG
CCCGATGCGTCATCAATTAGCCAGGAAGAACCACAGGGTGAAGCGACACCGGTAGGCA
ATAGAGAGTCTCCTGGACAAGCCGGAATGAACTCGATCCATTCCCCGGGCCCAGCATC
ACCGGTGAGCCACCCGGATGGCCAGGAGGCGAAGGCCCTGCCACCATTTGCCTGCGA
TGTTGGTGGTAGCGGTCATCATCACCATCATCATTAAAATGCAAAGCCCGCCGAAAGGC
GGGCTTTTCTGTACTTCGCAACGATTTCGGAGTCCGGAGACTCGCTGTTTTCGAAATTT
GCGCTCAAGGGCGGGTATTGAACC,843,CAATCCGCCCTCACTACAACCGGAGGTGACG
GGCCTCAAAGCCGCGTGGGAGTGAGCTAACACCGTGCGTGTTGACAATTTTACCTCTG
GCGGTGATAATGGTTGCAGCTACTAATAATTTTGTTTAACTTTAAGAAGGAGATATACCA
TGGGAAACGCTTATTACATTCAGGATCGTCTTGAGGCTCAGAGCTGGGCGCGTCACTAC
CAGCAGCTCGCCCGTGAAGAGAAAGAGGCAGAACTGGCAGACGACATGGAAAAAGGC
CTGCCCCAGCACCTGTTTGAATCGCTATGCATCGATCATTTGCAACGCCACGGGGCCA
GCAAAAAATCCATTACCCGTGCGTTTGATGACGATGTTGAGTTTCAGGAGCGCATGGCA
GAACACATCCGGTACATGGTTGAAACCATTGCTCACCACCAGGTTGATATTGATTCAGA
GGTAGGTGGTAGCGGTACCCCAAGCGCTTGTGTAGTCGAAAGAGAAGCAAGTACGCAC
AGCGGCAATCGGGGGGACGCCTTGAACTTGTCTTCTCCTAAAAGATCGAAGCCCGATG
CGTCATCAATTAGCCAGGAAGAACCACAGGGTGAAGCGACACCGGTAGGCAATAGAGA
GTCTCCTGGACAAGCCGGAATGAACTCGATCCATTCCCCGGGCCCAGCATCACCGGTG
AGCCACCCGGATGGCCAGGAGGCGAAGGCCCTGCCACCATTTGCCTGCGATGTTGGT
GGTAGCGGTCATCATCACCATCATCATTAAAATGCAAAGCCCGCCGAAAGGCGGGCTTT
TCTGTACTTCGCAACGATTTCGGAGTCCGGAGACTCGCTGTTTTCGAAATTTGCGCTCA
AGGGCGGGTATTGAACCCTACTCTGGCGTCGATGAGGGA
**SEQ ID NO:40 epitope from ZSCAN5A** 
TPSACVVEREASTHSGNRGDALNLSSPKRSKPDASSISQEEPQGEATPVGNRESPGQAGM
NSIHSPGPASPVSHPDGQEAKALPPFACDV
**SEQ ID NO:41 epitope from ZSCAN5B** 
SACVVEREALTHSGNRGDALNLSSPKRSKPDASSISQEEPQGEATPVGNRESPGQAEINPV
HSPGPAGPVSHPDGQEAKALPPFACDVC
**SEQ ID NO:42 epitope from ZSCAN5C** 
TPSACVVEREASTHSGSRGDALNLRGLKRSKPDATSISQEEPQGEATPVGNRESPGQAEIN
PVHSPGPAGPVSHPSGQEVKELLPFACEV
**SEQ ID NO:43 (Immunoglobulin heavy constant alpha 1)** 
ASPTSPKVFPLSLCSTQPDGNVVIACLVQGFFPQEPLSVTWSESGQGVTARNFPPSQDASG
DLYTTSSQLTLPATQCLAGKSVTCHVKHYTNPSQDVTVPCPVPSTPPTPSPSTPPTPSPSC
CHPRLSLHRPALEDLLLGSEANLTCTLTGLRDASGVTFTWTPSSGKSAVQGPPERDLCGCY
SVSSVLPGCAEPWNHGKTFTCTAAYPESKTPLTATLSKSGNTFRPEVHLLPPPSEELALNEL
VTLTCLARGFSPKDVLVRWLQGSQELPREKYLTWASRQEPSQGTTTFAVTSILRVAAEDWK
KGDTFSCMVGHEALPLAFTQKTIDRLAGKPTHVNVSVVMAEVDGTCY
**SEQ ID NO:44 (Immunoglobulin heavy constant alpha 2)** 
ASPTSPKVFPLSLDSTPQDGNVVVACLVQGFFPQEPLSVTWSESGQNVTARNFPPSQDAS
GDLYTTSSQLTLPATQCPDGKSVTCHVKHYTNSSQDVTVPCRVPPPPPCCHPRLSLHRPAL
EDLLLGSEANLTCTLTGLRDASGATFTWTPSSGKSAVQGPPERDLCGCYSVSSVLPGCAQ
PWNHGETFTCTAAHPELKTPLTANITKSGNTFRPEVHLLPPPSEELALNELVTLTCLARGFSP
KDVLVRWLQGSQELPREKYLTWASRQEPSQGTTTYAVTSILRVAAEDWKKGETFSCMVGH
EALPLAFTQKTIDRMAGKPTHINVSVVMAEADGTCY
**SEQ ID NO:45 (Immunoglobulin heavy constant delta)** 
APTKAPDVFPIISGCRHPKDNSPVVLACLITGYHPTSVTVTWYMGTQSQPQRTFPEIQRRDS
YYMTSSQLSTPLQQWRQGEYKCVVQHTASKSKKEIFRWPESPKAQASSVPTAQPQAEGSL
AKATTAPATTRNTGRGGEEKKKEKEKEEQEERETKTPECPSHTQPLGVYLLTPAVQDLWLR
DKATFTCFVVGSDLKDAHLTWEVAGKVPTGGVEEGLLERHSNGSQSQHSRLTLPRSLWNA
GTSVTCTLNHPSLPPQRLMALREPAAQAPVKLSLNLLASSDPPEAASWLLCEVSGFSPPNIL
LMWLEDQREVNTSGFAPARPPPQPRSTTFWAWSVLRVPAPPSPQPATYTCVVSHEDSRTL
LNASRSLEVSYVTDHGPMK
**SEQ ID NO:46 (Immunoglobulin heavy constant epsilon)** 
ASTQSPSVFPLTRCCKNIPSNATSVTLGCLATGYFPEPVMVTWDTGSLNGTTMTLPATTLTL
SGHYATISLLTVSGAWAKQMFTCRVAHTPSSTDWVDNKTFSVCSRDFTPPTVKILQSSCDG
GGHFPPTIQLLCLVSGYTPGTINITWLEDGQVMDVDLSTASTTQEGELASTQSELTLSQKHW
LSDRTYTCQVTYQGHTFEDSTKKCADSNPRGVSAYLSRPSPFDLFIRKSPTITCLVVDLAPS
KGTVNLTWSRASGKPVNHSTRKEEKQRNGTLTVTSTLPVGTRDWIEGETYQCRVTHPHLP
RALMRSTTKTSGPRAAPEVYAFATPEWPGSRDKRTLACLIQNFMPEDISVQWLHNEVQLPD
ARHSTTQPRKTKGSGFFVFSRLEVTRAEWEQKDEFICRAVHEAASPSQTVQRAVSVNPGK
**SEQ ID NO:47 (Immunoglobulin heavy constant gamma 1)**
**SEQ ID NO:48 (Immunoglobulin heavy constant gamma 2)** 
ASTKGPSVFPLAPCSRSTSESTAALGCLVKDYFPEPVTVSWNSGALTSGVHTFPAVLQSSG
LYSLSSVVTVPSSNFGTQTYTCNVDHKPSNTKVDKTVERKCCVECPPCPAPPVAGPSVFLF
PPKPKDTLMISRTPEVTCVVVDVSHEDPEVQFNWYVDGVEVHNAKTKPREEQFNSTFRVVS
VLTVVHQDWLNGKEYKCKVSNKGLPAPIEKTISKTKGQPREPQVYTLPPSREEMTKNQVSL
TCLVKGFYPSDISVEWESNGQPENNYKTTPPMLDSDGSFFLYSKLTVDKSRWQQGNVFSC
SVMHEALHNHYTQKSLSLSPGK
**SEQ ID NO:49 (Immunoglobulin heavy constant gamma 3 OS)** 
ASTKGPSVFPLAPCSRSTSGGTAALGCLVKDYFPEPVTVSWNSGALTSGVHTFPAVLQSSG
LYSLSSVVTVPSSSLGTQTYTCNVNHKPSNTKVDKRVELKTPLGDTTHTCPRCPEPKSCDT
PPPCPRCPEPKSCDTPPPCPRCPEPKSCDTPPPCPRCPAPELLGGPSVFLFPPKPKDTLMI
SRTPEVTCVVVDVSHEDPEVQFKWYVDGVEVHNAKTKPREEQYNSTFRVVSVLTVLHQDW
LNGKEYKCKVSNKALPAPIEKTISKTKGQPREPQVYTLPPSREEMTKNQVSLTCLVKGFYPS
DIAVEWESSGQPENNYNTTPPMLDSDGSFFLYSKLTVDKSRWQQGNIFSCSVMHEALHNR
FTQKSLSLSPGK
**SEQ ID NO:50 (Immunoglobulin heavy constant gamma 4)** 
ASTKGPSVFPLAPCSRSTSESTAALGCLVKDYFPEPVTVSWNSGALTSGVHTFPAVLQSSG
LYSLSSVVTVPSSSLGTKTYTCNVDHKPSNTKVDKRVESKYGPPCPSCPAPEFLGGPSVFL
FPPKPKDTLMISRTPEVTCVVVDVSQEDPEVQFNWYVDGVEVHNAKTKPREEQFNSTYRV
VSVLTVLHQDWLNGKEYKCKVSNKGLPSSIEKTISKAKGQPREPQVYTLPPSQEEMTKNQV
SLTCLVKGFYPSDIAVEWESNGQPENNYKTTPPVLDSDGSFFLYSRLTVDKSRWQEGNVFS
CSVMHEALHNHYTQKSLSLSLGK
**SEQ ID NO:51 (Immunoglobulin heavy constant mu)** 
GSASAPTLFPLVSCENSPSDTSSVAVGCLAQDFLPDSITFSWKYKNNSDISSTRGFPSVLRG
GKYAATSQVLLPSKDVMQGTDEHVVCKVQHPNGNKEKNVPLPVIAELPPKVSVFVPPRDGF
FGNPRKSKLICQATGFSPRQIQVSWLREGKQVGSGVTTDQVQAEAKESGPTTYKVTSTLTIK
ESDWLGQSMFTCRVDHRGLTFQQNASSMCVPDQDTAIRVFAIPPSFASIFLTKSTKLTCLVT
DLTTYDSVTISWTRQNGEAVKTHTNISESHPNATFSAVGEASICEDDWNSGERFTCTVTHTD
LPSPLKQTISRPKGVALHRPDVYLLPPAREQLNLRESATITCLVTGFSPADVFVQWMQRGQ
PLSPEKYVTSAPMPEPQAPGRYFAHSILTVSEEEWNTGETYTCVVAHEALPNRVTERTVDK
STGKPTLYNVSLVMSDTAGTCY
**SEQ ID NO:52 (Immunoglobulin lambda constant 1)** 
GQPKANPTVTLFPPSSEELQANKATLVCLISDFYPGAVTVAWKADGSPVKAGVETTKPSKQ
SNNKYAASSYLSLTPEQWKSHRSYSCQVTHEGSTVEKTVAPTECS
**SEQ ID NO:53 (Immunoglobulin lambda constant 2)** 
GQPKAAPSVTLFPPSSEELQANKATLVCLISDFYPGAVTVAWKADSSPVKAGVETTTPSKQ
SNNKYAASSYLSLTPEQWKSHRSYSCQVTHEGSTVEKTVAPTECS
**SEQ ID NO:54 (Immunoglobulin lambda constant 3)** 
GQPKAAPSVTLFPPSSEELQANKATLVCLISDFYPGAVTVAWKADSSPVKAGVETTTPSKQ
SNNKYAASSYLSLTPEQWKSHKSYSCQVTHEGSTVEKTVAPTECS
**SEQ ID NO:55 (Immunoglobulin lambda constant 6)** 
GQPKAAPSVTLFPPSSEELQANKATLVCLISDFYPGAVKVAWKADGSPVNTGVETTTPSKQ
SNNKYAASSYLSLTPEQWKSHRSYSCQVTHEGSTVEKTVAPAECS
**SEQ ID NO:56 (Immunoglobulin lambda constant 7)** 
GQPKAAPSVTLFPPSSEELQANKATLVCLVSDFNPGAVTVAWKADGSPVKVGVETTKPSKQ
SNNKYAASSYLSLTPEQWKSHRSYSCRVTHEGSTVEKTVAPAECS
**SEQ ID NO:57 (Immunoglobulin kappa constant)**

### Examples

### Summary

Methods: Four patients (P1-4) suffering from neurological conditions underwent serological investigation. For this purpose, patient sera were subjected to comprehensive autoantibody screening by indirect immunofluorescence assay (IIFA) with neuronal tissue sections or HEK293 cells expressing recombinant neuronal antigens. PhIP-Seq analysis with four sera displaying a similar fluorescence pattern on neuronal tissue IIFA was used to identify the autoantigen, which was verified by recombinant expression of the target antigen in HEK293 cells and use of the recombinant protein in immunoassays. Furthermore, sera or CSF of patients (n=68) with neurological symptoms and a similar reactivity on neuronal tissue sections compared to PS1-4 as well as healthy control sera (n=50) were analyzed in a recombinant cell based indirect immunofluorescence assay (RC-IIFA) with HEK293 cells expressing ZSCAN5B. 49 serum samples from patients with different neuronal autoantibodies (9 x anti-Neurochondrin positive, 10 x anti-Ri positive, 10 x anti-GAD65 positive, 10 x anti-Yo positive, 10 x anti-ITPR1 positive) were analyzed in RC-IIFA using ZSCAN5B but showed no positive reaction. Sera with positive reactions in RC-IIFA were analyzed by Western Blot with lysates of HEK293 cells recombinantly expressing ZSCAN5B and results were confirmed by Western Blot with recombinant ZSCAN5B purified from E. coli cells overexpressing ZSCAN5B. Furthermore, epitope containing protein fragments from ZSCAN5A, ZSCAN5B, and ZSCAN5C were produced in a cell free expression system and analyzed by Western Blot with the index sera.

Results: IIFA screening with patient sera (PS1-4) revealed IgG reactivity with the molecular layer and the granular layer in monkey cerebellum. Furthermore, no IgG reactivity was found with a panel of 30 recombinantly expressed established neural autoantigens. PS1-4 bound to peptides of ZSCAN5B and ZSCAN5C in PhIP-Seq analysis, and PS 1 and 3 bound in addition to peptides from ZSCAN5A. In RC-IIFA with ZSCAN5B expressing HEK293 cells all four patient sera showed a positive reaction which was not observed with 50 healthy control sera and with 49 control samples with other neuronal autoantibodies. In addition, 20 of the analyzed sera of 68 patients with similar fluorescence reactivity on neuronal tissue sections compared to PS1-4 were anti-ZSCAN5B positive. A positive reaction of patient sera but not control sera was also observed in immunoblot with recombinant ZSCAN5B-His. Similar Western Blot reactivities were observed with all index sera and epitope containing protein fragments from ZSCAN5A, ZSCAN5B and ZSCAN5C.

Clinical data of eight anti-ZSCAN5B positive patients were available. They suffered from encephalitis (3 x), paraneoplastic syndrome (1 x), dementia (1 x), neuromyelitis optica spectrum disorder (2 x), or neuromyotonia (1 x). In the case of the neuromyotonia diagnosis additional low titre anti-VGKC antibodies were detected by RIA during routine diagnostic workup.

### Methods

### Patients

All patient samples were sent to the Clinical Immunological Laboratory Stöcker, Lübeck (Germany) for the purpose of autoantibody testing. Subsequently, they were anonymized and provided to the authors. Control panels included sera of 50 healthy donors and 49 sera of patients having different neuronal autoantibodies.

### Indirect immunofluorescence assay (IFA)

IFA was performed using slides with a biochip mosaic of brain tissue cryosections (cerebellum of rat and monkey, hippocampus, thalamus of rat) in addition to recombinant HEK293 cells separately expressing 30 different neuronal antigens: Hu, Yo, Ri, CV2, PNMA2, ITPR1, Homer 3, CARP VIII, ARHGAP26, ZIC4, DNER/Tr, GAD65, GAD67, amphiphysin, recoverin, GABAB receptor, glycine receptor, DPPX, IgLON5, glutamate receptors (types NMDA, AMPA, mGluR1, mGluR5, GLURD2), LGI1, CASPR2, AQP4 (M1 and M23), MOG, ATP1A3, NCDN (EUROIMMUN, FA 111a-1003-51, FA 1112-1003-50, FA-1128-1003-50, FA112d-1003-1, FA 112m-1003-50, FA 1151-1003-50, Miske R, Hahn S, Rosenkranz T, Müller M, Dettmann IM, Mindorf S, Denno Y, Brakopp S, Scharf M, Teegen B, Probst C, Melzer N, Meinck HM, Terborg C, Stöcker W, Komorowski L., 2016, Autoantibodies against glutamate receptor δ2 after allogenic stem cell transplantation. Neurol Neuroimmunol Neuroinflamm., 3(4):e255; Scharf M, Miske R, Heidenreich F, Giess R, Landwehr P, Blöcker IM, Begemann N, Denno Y, Tiede S, Dähnrich C, Schlumberger W, Unger M, Teegen B, Stöcker W, Probst C, Komorowski L, 2015, Neuronal Na+/K+ ATPase is an autoantibody target in paraneoplastic neurologic syndrome, Neurology; 84(16):1673-9; Miske R, Gross CC, Scharf M, Golombeck KS, Hartwig M, Bhatia U, Schulte-Mecklenbeck A, Bönte K, Strippel C, Schöls L, Synofzik M, Lohmann H, Dettmann IM, Deppe M, Mindorf S, Warnecke T, Denno Y, Teegen B, Probst C, Brakopp S, Wandinger KP, Wiendl H, Stöcker W, Meuth SG, Komorowski L, Melzer N, 2016, Neurochondrin is a neuronal target antigen in autoimmune cerebellar degeneration, Neurol Neuroimmunol Neuroinflamm.;4(1):e307)). Each biochip array was incubated with serum or CSF diluted in PBS for 30 min, washed with PBS-Tween and immersed in PBS-Tween for 5 min. Subsequently, either fluorescein isothiocyanate (FITC)-labelled goat anti-human IgG (EUROIMMUN Medizinische Labordiagnostika AG, Luebeck Germany) or Alexa488-labelled goat anti-human IgG (1:500, Jackson Research, Suffolk, United Kingdom) were applied for 30 min. After another washing step with PBS-Tween, and embedding the slides in PBS-buffered DABCO-containing glycerol, they were analyzed by fluorescence microscopy. Based on the fluorescence intensity of the transfected cells in direct comparison with non-transfected cells and control samples, reactivities were classified as positive or negative. Endpoint titers refer to the last dilution showing visible fluorescence.

In competitive inhibition experiments, sera diluted 1:32 in PBS-Tween were preincubated for 1 h with recombinant ZSCAN5B expressing HEK293 cell lysate or as control empty vector transfected HEK293 cell lysate diluted 1:2 in PBS-Tween, before they were incubated in an IFA on neuronal tissue sections. Cell nuclei were visualized by DNA staining with TO-PRO-3 iodide (1:2000, ThermoFisher Scientific, Dreieich, Germany). Results were evaluated by two independent observers using laser scanning microscope (LSM700, Zeiss, Jena, Germany) or EUROStar II microscope (EUROIMMUN Medizinische Labordiagnostika AG, Lübeck, Germany). All incubation steps were carried out at room temperature.

### PhIP-Seq Analysis

PhIP-Seq analysis with four sera displaying a similar fluorescence pattern on neuronal tissue IIFA was used to identify the autoantibodies' target. PhIP-Seq analysis was performed at CDI Laboratories, Inc (Mayaguez, USA) using HuScan (Human proteome phage display) method with four patient sera (patient 1 - 4) showing a similar staining pattern in tissue indirect immunofluorescence assay (IIFA) on primate cerebellum and with 20 control sera showing different antibody binding patterns in neuronal tissue IIFA. A hit list displaying the results was generated.

### Recombinant expression of full length human ZSCAN5B and ZSCAN5B-His (Zinc finger and SCAN domain-containing protein 5B) in HEK293:

Two synthetic gene fragments coding for human ZSCAN5B were obtained from IDT (Integrated DNA Technologies, Inc.).

The first fragment is coding for amino acids 1-242 of human ZSCAN5B and the other one is coding for amino acids 243-495 of human ZSCAN5B including the Stop-codon. Both gene synthesis products were reamplified by PCR resulting in a 747 bp fragment-1 and a 793 bp fragment-2 (SEQ ID NO 25 and SEQ ID NO 26). Both PCR-fragments were Gel-purified and digested with Esp3I (New England Biolabs, R0734).

By PCR a third fragment of ZSCAN5B was amplified using the gene synthesis product (SEQ ID NO 26) as template and the DNA oligonucleotide primers sense ZSCAN5B-F3 (SEQ ID NO 27) and asense ZSCAN5B-F3 (SEQ ID NO 28), resulting in ZSCAN5B PCR fragment-3 without Stop-codon (SEQ ID NO 29). The amplification product was digested with Esp3I (New England Biolabs, R0734). The Esp3I-digested fragments of SEQ ID NO 25 and SEQ ID NO 26 were ligated with Ncol and Xhol linearized pTriEx-1 vector (Merck, Darmstadt, Germany) resulting in SEQ ID NO 30, coding for ZSCAN5B [human] (SEQ ID NO 31). The Esp3I-digested fragments of SEQ ID NO 25 and SEQ ID NO 29 were also ligated with Ncol and Xhol linearized pTriEx-1 vector (Merck, Darmstadt, Germany) resulting in SEQ ID NO 32, coding for ZSCAN5B [human] fused to a C-terminal octa Histidin-tag (H8) (SEQ ID NO 33).

ZSCAN5B-His or dHis were expressed in human HEK293 cells after ExGen500-mediated transfection (ThermoFisher Scientific, Schwerte, Germany) according to the manufacturer's instructions. In order to prepare substrates for IFA, HEK293 cells were seeded on sterile cover glasses, transfected, and allowed to express ZSCAN5B for 48 h. Cover glasses were washed with PBS, fixed with acetone for 10 min at room temperature, air-dried, cut into millimeter-sized biochips and used as substrates in IFA as described. Alternatively, cells were transfected in standard T-flasks and harvested after 5 days. The cell sediment was extracted with solubilization buffer. The extracts were stored in aliquots at -80 °C until further use. For purification of ZSCAN5B-His the recombinant antigen was expressed in E.coli cells and enriched by immobilized metal ion affinity chromatography combined with cation exchange chromatography.

### Cell-free protein expression of GamS-ZSCAN5A-6His, GamS-ZSCAN5B-6His & GamS-ZSCAN5C-6His:

Linear gene fragments of the three epitope-containing regions of ZSCAN5A, ZSCAB5B and ZSCAN5C were designed for the use in an E. coli cell-free protein synthesis system (myTXTL Linear DNA Expression Kit, arbor bioscience).

GamS, a 12kDa bacterial nuclease inhibitor, is well expressed in the "myTXTL Linear DNA Expression Kit" and was used as an N-terminal fusion partner to increase the molecular weight of each construct to facilitate detection by western blot. Further, a C-terminal 6His-Tag was added to each construct.

The resulting constructs GamS-ZSCAN5A-6His (SEQ ID NO 34), GamS-ZSCAN5B-6His (SEQ ID NO 35) and GamS-ZSCAN5C-6His (SEQ ID NO 36) were ordered from Twist Bioscience (South San Francisco, USA) with additional 3' and 5' DNA sequences, specific for the E.coli sigma70 promotor and terminator, required for the transcription and translation machinery of E. coli myTXTL linear DNA expression kit (GamS-ZSCAN5A-6His gene fragment with adapters (SEQ ID NO 37), GamS-ZSCAN5B-6His gene fragment with adapters (SEQ ID NO 38) & GamS-ZSCAN5C-6His_gene fragment with adapters (SEQ ID NO 39).

Protein expression was carried out according to the Daicel Arbor Biosciences "myTXTL Linear DNA Expression Kit" protocol (arbor bioscience). 9uL of cell-free expression mix in a 1,5mL Eppendorf tube was mixed with 3uL of 80 nM dsDNA and incubated at 29°C for 16h. After incubation 400 µL wash buffer (20mM HEPES, pH8, 300 mM NaCl, 20mM Imidazol) and 30 µL magnetic His-beads (Cube BiotechBiocube, Monheim, Germany) were added to each expression mix and incubated for 30min. Magnetic beads were washed twice in washing buffer in a magnetic holder. Proteins were eluted in 100 µl elution buffer (20mM HEPES, pH8, 300 mM NaCl, 500mM Imidazol) and subsequently buffer exchanged into PBS with spin desalting colums (zeba, ThermoFisher Scientific, Schwerte, Germany).

### Immunoblot

HEK-ZSCAN5B-His fractions, fractions from ZSCAN5B-His purified from E. coli or ZSCAN-6His fractions were separated by SDS-PAGE (NuPAGE, ThermoFisher Scientific, Schwerte, Germany) and subsequently electrotransferred onto a nitrocellulose membrane by tank blotting with transfer buffer (ThermoFisher Scientific, Schwerte, Germany) according to the manufacturer's instructions. The membranes were blocked with 1:5 diluted Blot Wash Buffer Casein (EUROIMMUN, Germany) for 15 min and incubated with diluted patient or control sera (dilution 1:200) in Blot Wash Buffer Casein for 3 h, followed by 3 washing steps with Universal Blot Buffer (EUROIMMUN Medizinische Labordiagnostika AG, Lübeck), a second incubation for 30 min with anti-human-IgG-AP (EUROIMMUN Medizinische Labordiagnostika AG, Lübeck), 3 washing steps, and staining with NBT/BCIP substrate (EUROIMMUN Medizinische Labordiagnostika AG, Lübeck). Reagents were obtained from Merck, Darmstadt, Germany or Sigma-Aldrich, Heidelberg, Germany if not specified otherwise.

### Results

### Characterization of the patients' autoantibodies

Indirect immunofluorescence assays (IIFA) using permeabilized cerebellar cryosections from rat and primate and the sera of patients 1-4 (PS1-4) showed a spotty staining of the granular layer and a granular staining of the molecular layer (Fig. 1). Further monospecific analyses with recombinant HEK293 cells expressing 30 neural autoantigens (Hu, Yo, Ri, CV2, PNMA2, SOX1, ITPR1, Homer 3, CARP VIII, ARHGAP26, ZIC4, DNER/Tr, GAD65, GAD67, amphiphysin, recoverin, GABAB receptor, glycine receptor, DPPX, IgLON5, glutamate receptors [types NMDA, AMPA, mGluR1, mGluR5, GLURD2], LGI1, CASPR2, AQP4 [M1 and M23], MOG, ATP1A3, and NCDN) revealed no positive reactions.

### Identification of ZSCAN5B, ZSCAN5C, and ZSCAN5A as the target of neuronal autoantibodies

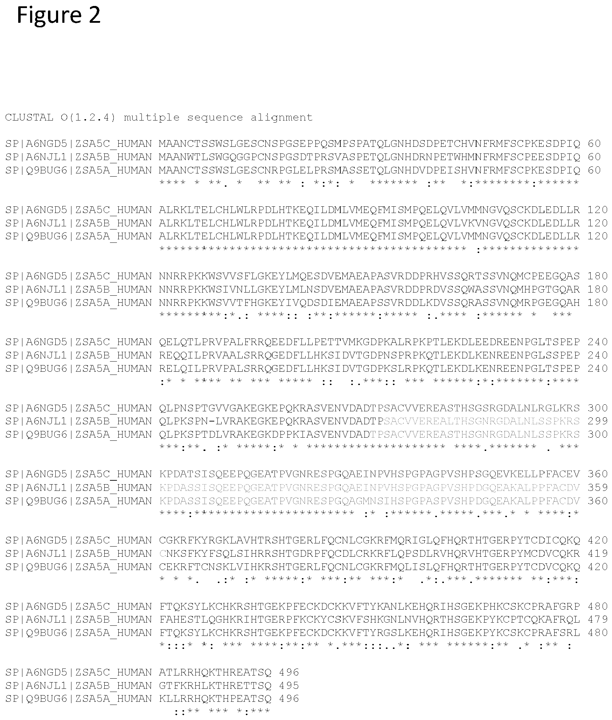

Patient sera 1-4 bound to peptides of human ZSCAN5B (Zinc finger and SCAN domain-containing protein 5B, Uniprot acc. # A6NJL1) and ZSCAN5C (Zinc finger and SCAN domain-containing protein 5C, Uniprot acc. # A6NGD5) in PhlP-Seq analysis. PS 1 and 3 bound in addition to peptides from ZSCAN5A (Zinc finger and SCAN domain-containing protein 5A, Uniprot acc. # Q9BUG6) (Tab. 1). None of the 20 control sera, showing different staining patterns in neuronal tissue IIFA, bound to peptides of one of the three proteins ZSCAN5B, ZSCAN5C, or ZSCAN5A. All four patient sera bound to the same peptide of ZSCAN5B as well as to a peptide from a homologous region in ZSCAN5C and ZSCAN5A (Fig. 2) indicating the epitope detected by the patient sera might be located to amino acid residues 272 -360 in ZSCAN5B and amino acid residues 271 -360 in ZSCAN5C and ZSCAN5A.

**Table 1. PhlP-Seq analysis results.**

| PhIP-Seq analysis results for ZSCAN5A, ZSCAN5B, and ZSCAN5C are summarized for serum samples of patient 1 - 4. HuScan Scores and hit-numbers for all four index patient samples are given. | | | | | | |
|---|---|---|---|---|---|---|
| Sample | PhiP-Seq Score | | | Hit No. | | |
| | ZSCAN5A Uniprot acc. # Q9BUG6 | ZSCAN5B Uniprot acc. # A6NJL1 | ZSCAN5C Uniprot acc. # A6NGD5 | ZSCAN5A Uniprot acc. # Q9BUG6 | ZSCAN5B Uniprot acc. # A6NJL1 | ZSCAN5C Uniprot acc. # A6NGD5 |
| Patient 1 | 25,97 | 106,97 | 106,07 | 52 | 3 | 4 |
| Patient 2 | - | 11,03 | 10,89 | - | 54 | 56 |
| Patient 3 | 28,29 | 101,49 | 97,93 | 17 | 1 | 2 |
| Patient 4 | - | 7,45 | 7,36 | - | 114 | 118 |

### Neutralization of neuronal tissue staining pattern

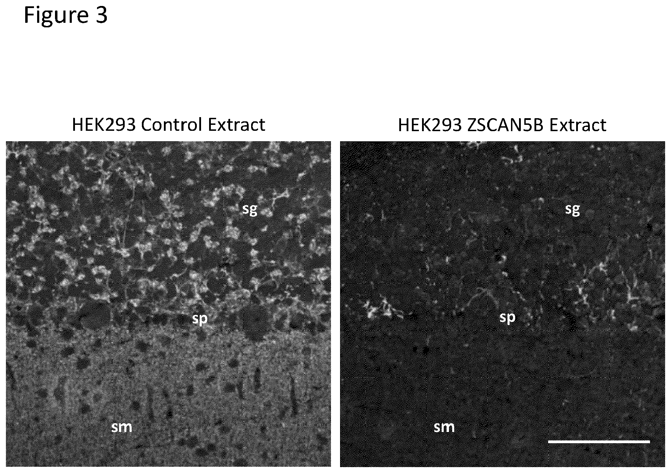

The neuronal tissue IIFA reaction of the patients' autoantibodies could be abolished by preincubation of serum of patient 3 with HEK293 lysate containing ZSCAN5B-His (SEQ ID NO 10) (Fig. 3).

### Detection of anti-ZSCAN5A, anti-ZSCAN5B, and anti-ZSCAN5C autoantibodies in immunoassays with recombinant full length ZSCAN5B and epitope containing protein fragments of ZSCAN5A, ZSCAN5B, and ZSCAN5C

In recombinant cell based IIFA (RC-IIFA) with HEK293-ZSCAN5B and HEK293-ZSCAN5B-His cells (SEQ ID NO 8 and SEQ ID NO 10), PS1-4 reacted with HEK293-ZSCAN5B and HEK293-ZSCAN5B-His (endpoint titer 1:3200, 1:1000, 1:10, 1:3200) but not with empty vector transfected HEK cells (Fig. 4). Furthermore, 20 out of 68 patient sera, which displayed a similar reactivity in IFA with neuronal tissue sections as PS1-4, revealed positive reactions in RC-IIFA with HEK293-ZSCAN5B and HEK293-ZSCAN5B-His cells, whereas none of 50 sera from healthy donors showed a similar reactivity (1:10 dilution). Positive reactions were also not observed with sera from 49 patients with different neuronal autoantibodies (9 x anti-Neurochondrin positive, 10 x anti-Ri positive, 10 x anti-GAD65 positive, 10 x anti-Yo positive, 10 x anti ITPR1-positive). CSF was available from 2 patients with positive serum reactions in RC-IIFA. Both CSF were negative in neuronal tissue IIFA but showed positive reactions in RC-IIFA with HEK293-ZSCAN5B cells (endpoint titer 1:1 or 1:3,2).

Immunoblot was performed using recombinant HEK293-ZSCAN5B-His lysate and results were confirmed using ZSCAN5B-His purified from E. coli. Expression of the recombinant 57 kDa ZSCAN5B-His protein was demonstrated using anti-His-tag antibody. Incubation of 13 patient sera and 15 healthy control sera revealed a strong reaction of all 13 patient sera with the recombinant protein, whereas none of the 15 healthy control sera showed a similar reaction (Fig. 5A).

Clinical data were available of patient 4-11 and are summarized in Tab. 2. In addition, epitope-containing protein fragments of ZSCAN5A, ZSCAN5B and ZSCAN5C were each analyzed with patient sera PS 1-4 and with anti-His Tag antibody as control. All four analyzed patient sera detected GamS-ZSCAN5A (271-360)-His, GamS-ZSCAN5B (272-360)-His, and GamS-ZSCAN5C (271-360)-His with similar intensities (Fig. 5B).

**Table 2. Clinical characterization.**

| Clinical information was available for eight patients. Serum endpoint titers are listed. From one patient also CSF was available. | | | |
|---|---|---|---|
| Patient No. | Sex, Age | ZSCAN5B RC-IIFA Endpoint titer (serum) | Clinical information |
| 4 | F, 27 | 1:3200 | Neuromytonia |
| 5 | F, 15 | 1:1000 | NMOSD suspected |
| 6 | F, 71 | 1:3200 | Autoimmune encephalitis |
| 7 | M, 57 | 1:1000 | Encephalitis |
| 8 | M, 51 | 1:320 | NMOSD or MS suspected |
| 9 | F, 49 | 1:320 (serum); 1:1 (CSF) | Limbic encephalitis |
| 10 | M, 64 | 1:100 | Paraneoplastic syndrome |
| 11 | F, 81 | 1:32 | Dementia |

Using IIFA, autoantibodies to ZSCAN5B were found in 37 additional patients, several of whom presented with at least one of epilepsy, encephalitis such as autoimmune encephalitis and limbic encephalitis, dementia, cognitive decline, ataxia, neuropathy and paraneoplastic syndrome.

## Claims

1. A method comprising the step detecting in a sample a mammalian antibody binding specifically to ZSCAN5.

2. A method for isolating a mammalian antibody binding specifically to ZSCAN5, comprising the steps
a) immobilizing on a carrier at least one polypeptide comprising at least one epitope of ZSCAN5 or a variant thereof,
b) contacting a sample comprising antibodies with the at least one polypeptide under conditions compatible with formation of a complex, wherein said antibody binds to said polypeptide,
c) separating the complex formed in step a) from the sample,
d) optionally separating the antibody from the polypeptide, and
e) optionally detecting the antibody or complex.

3. A method comprising
contacting a polypeptide comprising at least one epitope of ZSCAN5 or a variant thereof with a liquid comprising a mammalian antibody binding specifically to ZSCAN5, wherein a candidate drug is present and/or the liquid does not comprise a sample from a subject to be diagnosed, and
detecting binding of the antibody, preferably in quantitative manner.

4. A mammalian antibody binding specifically to ZSCAN5, preferably in a liquid comprising one or more, more preferably all from the group comprising an artificial buffer, a preservative and an artificial anticoagulant and/or from the group comprising blood such as whole blood, plasma, serum and CSF, optionally wherein the liquid is diluted.

5. A carrier comprising a solid phase comprising at least one immobilized polypeptide comprising at least one epitope of ZSCAN5 or a variant thereof and a) a negative and/or positive control and/or b) at least one additional diagnostically useful antigen, wherein the at least one immobilized polypeptide and the negative and/or positive control or additional antigen are spatially separate on the carrier,
or a first carrier with a solid phase comprising at least one immobilized polypeptide comprising at least one epitope of ZSCAN5 or a variant thereof and a second carrier comprising a solid phase comprising an immobilized a) negative and/or positive control and/or b) at least one additional polypeptide comprising an immobilized antigen or a variant thereof.

6. The carrier according to claim 5, wherein the at least one additional antigen is at least one antigen, preferably all antigens from the group consisting of NMDAR, Lgl1, AMPA1, AMPA2, CASPR2, GABA B, GABA A, DPPX, IGLON5, Hu, Yo, CV2/CRMP5, Ri, Ma2, Amphiphysin, Recoverin, RGS8, DAGLA, STX1B, AK5, AP3B2, Flotillin1+2, GRM1, GRM2, GRM5, GLURD2, ITPR1, KCNA2, NCDN, Septin 3+5+6+7+11, Sez6L2, or a variant thereof.

7. A kit comprising at least one polypeptide comprising at least one epitope of ZSCAN5 or a variant thereof and a carrier, preferably according to any one of claims 5 to 6, wherein
a) the at least one polypeptide is immobilized on a carrier, preferably the carrier according to any one of claims 5 to 6, and the kit further comprises a means for detecting a mammalian antibody,
b) the at least one polypeptide and the carrier are configured for immobilizing the at least one polypeptide on the surface of the carrier, preferably *via* an affinity tag and a ligand binding to the affinity tag, and the kit further comprises a means for detecting a mammalian antibody,
c) the carrier is immobilized with a means for capturing an antibody and the kit further comprises a means for detecting a mammalian antibody, preferably the at least one polypeptide comprising a detectable label, or
d) the carrier and a means for capturing an antibody are configured for immobilizing the means for capturing an antibody on the surface of the carrier, preferably *via* an affinity tag and a ligand binding to the affinity tag, and the kit further comprises a means for detecting a mammalian antibody, preferably the at least one polypeptide comprising a detectable label,
and preferably one or more, more preferably all from the group consisting of a recombinant antibody binding to ZSCAN5, a mammalian antibody binding to ZSCAN5, a chemical solution reactive with a detectable label, a positive control, a negative control, a water-tight vessel for incubating a sample with a carrier or reagent, a sample dilution buffer, a wash buffer, and a calibrator, preferably a set of calibrators.

8. A use of
a) a mammalian antibody binding specifically to ZSCAN5 or a variant thereof; or
b) the combination of at least one polypeptide comprising at least one epitope of ZSCAN5 or a variant thereof and a means for detecting a mammalian antibody; or
c) the carrier according to any one of claims 5 to 6; or
d) the kit according to claim 7
for identifying a patient having an increased risk of suffering from or developing a disease associated with the presence of a mammalian antibody binding specifically to ZSCAN5.

9. A use of at least one polypeptide comprising at least one epitope of ZSCAN5 or a variant thereof, or a mammalian antibody binding specifically to ZSCAN5 or a variant thereof, or a recombinant antibody binding specifically to ZSCAN5 or a variant thereof for the manufacture of an analytical kit or medical device.

10. A use of a mammalian antibody binding specifically to ZSCAN5 or a variant thereof, or a recombinant antibody binding specifically to ZSCAN5 or a variant thereof, wherein the antibody is preferably recognized by secondary antibodies to mammalian IgG class immunoglobulins, as a positive control for the detection of an antibody binding specifically to ZSCAN5 or a variant thereof in a sample.

11. An *ex vivo* method for removing a mammalian antibody binding specifically to ZSCAN5 or a variant thereof from the blood of a mammalian patient.

12. The method according to any one of claims 2-3, the carrier according to any one of claims 5-6, the kit according to claim 7 or the use according to claim 8 or 9, wherein the polypeptide is a recombinant, isolated and/or purified polypeptide.

13. The method according to any one of claims 1-3 and 12, the kit according to claim 7 or 12 or the use according to any one of claims 8-10 and 12, wherein the sample is selected from the group comprising whole blood, plasma, serum, cerebrospinal fluid and saliva.

14. The method according to any one of claims 1-3 and 11-13, the kit according to any one of claims 7 and 12-13 or the use according to any one of claims 8-10 and 12-13, wherein the antibody or complex is detected using a detection method selected from the group consisting of immunodiffusion, electrophoresis, light scattering immunoassays, agglutination, labeled immunoassays such as those from the group comprising radiolabeled immunoassay, enzyme immunoassays, more preferably ELISA, chemiluminescence immunoassays, preferably electrochemiluminescence immunoassay, and immunofluorescence, preferably indirect immunofluorescence.

15. The method according to any one of claims 1-3 and 12-14, the carrier according to any one of claims 5-6 and 12, the kit according to any one of claims 7 and 12-14 or the use according to any one of claims 8-10 and 12-14, wherein the carrier is selected from the group consisting of a glass slide, preferably for microscopy, a biochip, a microtiter plate, a lateral flow device, a test strip, a membrane, preferably a line blot, a chromatography column and a bead, preferably a magnetic or fluorescent bead.
